# EUROPEAN PATENT SPECIFICATION

(11) **EP 2 728 003 B1**
(45) Date of publication and mention of the grant of the patent: **27.12.2017**
(21) Application number: 12805134.9
(22) Date of filing: 29.06.2012
(51) Int. Cl.: C12N 15/09, C12Q 1/04, C12Q 1/68, G01N 33/50

(54) **METHOD FOR DIAGNOSING TYPE OF PANCREATIC TUMOR**
VERFAHREN ZUR DIAGNOSE VON PANKREASTUMOREN
MÉTHODE DE DIAGNOSTIC DU TYPE DE TUMEUR PANCRÉATIQUE

(30) Priority: 29.06.2011 JP 2011144847
(43) Date of publication of application: 07.05.2014
(73) Proprietor: Kagoshima University, Kagoshima-shi, Kagoshima 890-8580 (JP)
(72) Inventor: YOKOYAMA, Seiya, Kagoshima-shi Kagoshima 890-8580 (JP); YONEZAWA, Suguru, Kagoshima-shi Kagoshima 890-8580 (JP); KITAMOTO,Sho, Kagoshima-shi Kagoshima 890-8580 (JP)
(74) Representative: Vossius & Partner Patentanwälte Rechtsanwälte mbB
(86) International application number: PCT/JP2012/067262
(87) International publication number: WO 2013/002422

(56) References cited:
- WO-A1-2011/099566
- WO-A1-2011/132798
- SUGURU YONEZAWA ET AL: "Significance of mucin expression in pancreatobiliary neoplasms", JOURNAL OF HEPATO-BILIARY-PANCREATIC SCIENCES ; (FORMERLY: JOURNAL OF HEPATO-BILIARY-PANCREATIC SURGERY), SPRINGER JAPAN, JAPAN, vol. 17, no. 2, 29 September 2009 (2009-09-29), pages 108-124, XP019804353, ISSN: 1868-6982
- SEIYA YOKOYAMA ET AL: "Diagnosis of Pancreatic Neoplasms Using a Novel Method of DNA Methylation Analysis of Mucin Expression in Pancreatic Juice", PLOS ONE, vol. 9, no. 4, 8 April 2014 (2014-04-08), page e93760, XP055169149, DOI: 10.1371/journal.pone.0093760
- SUGURU YONEZAWA: 'Mucin gene expression mechanism related to a new classification of pancreatobiliary neoplasms' 27 May 2011, XP055141997 Retrieved from the Internet: <URL:http://ir. kagoshima-u.ac.jp/bitstream/10232/11993/1/ 20590345seika.pdf> [retrieved on 2012-09-04]
- FURUKAWA TORU ET AL.: 'Classification of types of intraductal papillary-mucinous neoplasm of the pancreas: a consensus study.' VIRCHOWS ARCHIV. vol. 447, no. 5, 2005, pages 794 - 799, XP035106194
- YONEZAWA SUGURU ET AL.: 'Precursor lesions of pancreatic cancer.' GUT AND LIVER vol. 2, no. 3, 2008, pages 137 - 154, XP055142115
- YOSHIHISA NAITO ET AL.: 'Histopathological and Immunohistochemical Study of Intraductal Papillar y Mucinous Neoplasm of the Pancreas with Special Reference to Its Histologic Subtypes' JOURNAL OF SAITAMA MEDICAL UNIVERSITY vol. 35, no. L, December 2008, pages T9 - T22, XP008172324
- ABRAMS, EZRA S. ET AL.: 'Comprehensive detection of single base changes in human genomic DNA using denaturing gradient gel electrophoresis and a GC clamp.' GENOMICS vol. 7, no. 4, 1990, pages 463 - 475, XP024864895
- SUGURU YONEZAWA: 'Mucin: Hito Gan ni Okeru Rinsho Byorigakuteki Igi, Idenshi Hatsugen Kiko, Soki Shindan System eno Oyo' JOURNAL OF THE JAPANESE SOCIETY OF CLINICAL CYTOLOGY vol. 49, no. 2, 22 September 2010, page 431, XP008172836
- YASUHIRO HIBI ET AL.: 'Pancreatic juice cytology and subclassification of intraductal papillary mucinous neoplasms of the pancreas' THE JOURNAL OF JAPAN PANCREAS SOCIETY vol. 22, no. 4, 2007, pages 519 - 520, XP008172345
- SUGURU YONEZAWA: 'Mucin: Hito Gan ni Okeru Rinsho Byorigakuteki Igi to Idenshi Hatsugen Kiko no Kaimei kara Shuyo Akuseido Soki Shindan System no Kochiku made' NIPPON BYORI GAKKAI KAISHI vol. 99, 26 March 2010, pages 121 - 122, XP008172838
- SUGURU YONEZAWA ET AL.: 'Mucin kara Mita Shuyo Shinten Process' KANTANSUI vol. 58, no. 6, 28 June 2009, pages 755 - 767, XP008172839
- SUGURU YONEZAWA ET AL.: 'IPMN no Soshiki Agata to Bunka Hatsuiku' KANTANSUI vol. 61, no. 3, 28 September 2010, pages 343 - 358, XP008172840
- MAKER, AJAY V. ET AL.: 'Pancreatic cyst fluid and serum mucin levels predict dysplasia in intraductal papillary mucinous neoplasms of the pancreas.' ANNALS OF SURGICAL ONCOLOGY. vol. 18, no. 1, January 2011, pages 199 - 206, XP019872263

## Description

### Technical Field

The present description generally relates to a method for diagnosis of pancreatic tumor type utilizing, for example, a pancreatic fluid sample. The present invention relates to an examination method for determining pancreatic tumor type comprising:
a first step of detecting the degree of methylation in a 5'-untranslated region or a region comprising a 5'-untranslated region and a translated region of a gene encoding a mucin core protein in a set of genes including the MUC1 gene, the MUC2 gene, the MUC4 gene, and the MUC5AC gene in a pancreatic fluid sample from a subject, wherein the 5'-untranslated region or the region comprising a 5'-untranslated region and a translated region in each of the MUC1 gene, the MUC2 gene, the MUC4 gene, and the MUC5AC gene comprises the nucleotide sequence as shown in SEQ ID NOs: 1 to 4, respectively; and
a second step of identifying a pancreatic tumor, using the degree of methylation as an index value, as being of pancreatic tumor type selected from the group consisting of pancreatic ductal adenocarcinoma, an intraductal papillary mucinous neoplasm of the gastric type, and an intraductal papillary mucinous neoplasm of the intestinal type, wherein the methylated or unmethylated statuses of the genes are evaluated by designating cut-off values for methylated DNA content as 50% for the MUC1 gene, 70% for the MUC2 gene, 50% for the MUC4 gene and 60% for the MUC5AC gene and, the pancreatic tumor is identified as:
   (a) an intraductal papillary mucinous neoplasm of the gastric type when the 5'-untranslated regions or the regions comprising 5'-untranslated regions and translated regions of the MUC1 gene, the MUC2 gene, the MUC4 gene, and the MUC5AC gene are methylated;
   (b) an intraductal papillary mucinous neoplasm of the intestinal type when the 5'-untranslated regions or the regions comprising 5'-untranslated regions and translated regions of the MUC1 gene, the MUC2 gene, the MUC4 gene, and the MUC5AC gene are unmethylated; or
   (c) a pancreatic ductal adenocarcinoma when the 5'-untranslated regions or the regions comprising 5'-untranslated regions and translated regions of the MUC1 gene, the MUC2 gene, and the MUC4 gene are unmethylated and the 5'-untranslated region or the region comprising a 5'-untranslated region and a translated region of the MUC5AC gene is methylated.

### Background Art

Most alimentary canal cancers, such as gastric cancer and colon cancer, have become detectable at an early stage and curable due to the development of techniques such as x-ray contrast radiography and endoscopy. Thus, such cancers can be overcome by establishing social systems that take care of burdens and expenses for undergoing medical examinations imposed on individuals; that is, most alimentary canal cancers are "under the control of humans."

In the case of pancreatic cancer, however, in many cases, neither diagnosis nor even early detection is possible at a curable stage, even if state-of-the art equipment, such as PET-CT, is used, and pancreatic cancer is a representative example of refractory cancer. Even if a tumor is detected via diagnostic imaging, pathological and qualitative diagnosis thereof via biopsy or other means remains difficult. Thus, such pancreatobiliary tumors are characterized by the difficulty of determining whether or not the detected tumor is "a high-grade malignant tumor in urgent need of highly-invasive surgery" or "a benign lesion to be followed upon." Accordingly, development of methods for early detection of pancreatic cancer and diagnosis of pancreatic cancer type has been awaited.

Meanwhile, methods for cancer diagnosis comprising detection and analysis of DNA methylation are generally known.

For example, Patent Document 1 discloses a method of determining the predisposition of a subject to the development of a cell proliferation or neoplastic disorder comprising a step of analyzing a biological sample for a change in methylation status or a polymorphism of a target gene, such as the H19 gene or IGF2 gene.

Patent Document 2 discloses a method for diagnosing cancer comprising the detection of a methylated SPARC nucleic acid molecule or a variant thereof in a sample from a subject.

Patent Document 3 discloses a method for detecting pancreatic cancer in a subject comprising bringing a nucleic-acid-containing specimen from the subject into contact with an agent that allows determination of the methylation state of at least one gene or an associated regulatory region of such a gene, identifying aberrant methylation of regions of the gene or regulatory region, and detecting pancreatic cancer in the subj ect.

Patent Documents 4 to 11 each disclose a method of evaluating the degree of canceration of a mammal-origin specimen comprising: a step of measuring a methylation frequency of a target gene contained in a mammal-origin specimen or an index value having a correlation therewith; and a step of determining the cancerous state of the specimen based on the difference obtained by comparing the measured methylation frequency or an index value having correlation therewith with a control. In Patent Documents 4 to 11, examples of target genes include the disintegrin and metalloproteinase domain 23 gene, the HAND1 gene, the Solute carrier family 6 neurotransmitter transporter noradrenalin member 2 gene, the G-protein coupled somatostatin and angiotensin-like peptide receptor gene, the G protein-coupled receptor 7 gene, the Neurofilament 3 gene, the Fibrillin 2 gene, and the p53-responsive gene 2 gene.

Yonezawa et al., Journal of Hepato-Biliary-Pancreatic Sciences 17(2) (2009), 108-124 describes differential expression of different mucin genes in different pancreas tumour types and stages. However, the Yonezawa publication does not disclose or suggest that a different methylation pattern of the four mucin genes MUC1, MUC2, MUC4 and MUC5 can be used as a tool for the determination of pancreatic cancer types in a pancreatic fluid sample.

In the past, however, it was not known that pancreatic tumor type could be diagnosed by detecting the degree of methylation of a particular gene or a regulatory region thereof.

### Prior Art Documents

### Patent Documents

Patent Document 1: JP 2008-504018 A
Patent Document 2: JP 2007-524393 A
Patent Document 3: JP 2007-524369 A
Patent Document 4: JP 2005-110645 A
Patent Document 5: JP 2004-135661 A
Patent Document 6: JP 2005-087050 A
Patent Document 7: JP 2005-087049 A
Patent Document 8: JP 2005-087048 A
Patent Document 9: JP 2005-087047 A
Patent Document 10: JP 2005-087046 A
Patent Document 11: JP 2005-087045 A

### Summary of the Invention

Under the above circumstances, it is an object of the present invention to provide a method that allows early diagnosis of pancreatic tumor type.

Concentrated studies were conducted in order to attain the above object. As a result, it was discovered that pancreatic tumor type could be diagnosed by detecting the degree of methylation in a 5'-untranslated region or a region comprising a 5'-untranslated region and a translated region of a gene encoding a mucin core protein in a pancreatic fluid sample from a subject. This has led to the completion of the present invention.

The present invention includes the following features.
(1) An examination method for determining pancreatic tumor type comprising:
   a first step of detecting the degree of methylation in a 5'-untranslated region or a region comprising a 5'-untranslated region and a translated region of a gene encoding a mucin core protein in a set of genes including the MUC1 gene, the MUC2 gene, the MUC4 gene, and the MUC5AC gene in a pancreatic fluid sample from a subject, wherein the 5'-untranslated region or the region comprising a 5'-untranslated region and a translated region in each of the MUC1 gene, the MUC2 gene, the MUC4 gene, and the MUC5AC gene comprises the nucleotide sequence as shown in SEQ ID NOs: 1 to 4, respectively;
   and a second step of identifying a pancreatic tumor, using the degree of methylation as an index value, as being of pancreatic tumor type selected from the group consisting of pancreatic ductal adenocarcinoma, an intraductal papillary mucinous neoplasm of the gastric type, and an intraductal papillary mucinous neoplasm of the intestinal type, wherein the methylated or unmethylated statuses of the genes are evaluated by designating cut-off values for methylated DNA content as 50% for the MUC1 gene, 70% for the MUC2 gene, 50% for the MUC4 gene and 60% for the MUC5AC gene and,_the pancreatic tumor is identified as:
      (a) an intraductal papillary mucinous neoplasm of the gastric type when the 5'-untranslated regions or the regions comprising 5'-untranslated regions and translated regions of the MUC1 gene, the MUC2 gene, the MUC4 gene, and the MUC5AC gene are methylated;
      (b) an intraductal papillary mucinous neoplasm of the intestinal type when the 5'-untranslated regions or the regions comprising 5'-untranslated regions and translated regions of the MUC1 gene, the MUC2 gene, the MUC4 gene, and the MUC5AC gene are unmethylated; or
      (c) a pancreatic ductal adenocarcinoma when the 5'-untranslated regions or the regions comprising 5'-untranslated regions and translated regions of the MUC1 gene, the MUC2 gene, and the MUC4 gene are unmethylated and the 5'-untranslated region or the region comprising a 5'-untranslated region and a translated region of the MUC5AC gene is methylated.
(2) The method according to (1), wherein the first step comprises steps of:
   subjecting DNA obtained from a pancreatic fluid sample to bisulfite treatment;
   subjecting the DNA after bisulfite treatment to a first PCR using a first set of primers corresponding to outer regions of a 5'-untranslated region or a region comprising a 5'-untranslated region and a translated region of the gene encoding a mucin core protein;
   subjecting the DNA amplified via the first PCR to a second PCR using a second set of primers corresponding to the 5'-untranslated region or the region comprising a 5'-untranslated region and a translated region of the gene encoding a mucin core protein;
   and subjecting the DNA amplified via the second PCR to denaturing gradient gel electrophoresis, with the annealing positions of the second set of primers being located inside the annealing positions of the first set of primers relative to the 5'-untranslated region or the region comprising a 5'-untranslated region and a translated region of the gene encoding a mucin core protein.
(3) The method according to (2), wherein one of a pair of primers constituting the second set of primers has a GC-clamp sequence in its 5' side.
(4) The method according to (2) or (3), wherein the density gradient of the denaturing gradient gel is limited to a denaturing density gradient.
(5) Use of an examination kit for implementing a method according to any one of (1) to (4) for determining pancreatic tumor type, comprising:
   a set of primers used for implementing the method according to any one of claims 1 to 4; and an instruction for determining pancreatic tumor type describing identification of a pancreatic tumor as:
      (a) an intraductal papillary mucinous neoplasm of the gastric type when the 5'-untranslated regions or the regions comprising 5'-untranslated regions and translated regions of the MUC1 gene, the MUC2 gene, the MUC4 gene, and the MUC5AC gene are methylated;
      (b) an intraductal papillary mucinous neoplasm of the intestinal type when the 5'-untranslated regions or the regions comprising 5'-untranslated regions and translated regions of the MUC1 gene, the MUC2 gene, the MUC4 gene, and the MUC5AC gene are unmethylated; or
      (c) a pancreatic ductal adenocarcinoma when the 5'-untranslated regions or the regions comprising 5'-untranslated regions and translated regions of the MUC1 gene, the MUC2 gene, and the MUC4 gene are unmethylated and the 5'-untranslated region or the region comprising a 5'-untranslated region and a translated region of the MUC5AC gene is methylated.
(6) The use according to (5), which comprises a first set of primers corresponding to outer regions of the 5'-untranslated region or the region comprising a 5'-untranslated region and a translated region of a gene encoding a mucin core protein and a second set of primers corresponding to the 5'-untranslated region or the region comprising a 5'-untranslated region and a translated region of a gene encoding a mucin core protein, with the annealing positions of the second set of primers being located inside the annealing positions of the first set of primers relative to the 5'-untranslated region or a region comprising a 5'-untranslated region and a translated region of a gene encoding a mucin core protein.
(7) The use according to (5) or (6), which comprises a set of primers consisting of the nucleotide sequences as shown in SEQ ID NOs: 14 to 29, respectively.

### Brief Description of the Drawings

Fig. 1A shows the sequence of a promoter region that regulates human mucin core protein 1 (MUC1) gene expression through methylation and the sequence of a translated region adjacent thereto (SEQ ID NO: 5).
Fig. 1B shows the sequence of a promoter region that regulates human MUC1 gene expression through methylation and the sequence of a translated region adjacent thereto (SEQ ID NO: 6; the DNA sequence after bisulfite treatment, with uracil converted from unmethylated cytosine being indicated as "thymine").
Fig. 2A shows the sequence of a promoter region that regulates human mucin core protein 2 (MUC2) gene expression through methylation and the sequence of a translated region adjacent thereto (SEQ ID NO: 7).
Fig. 2B shows the sequence of a promoter region that regulates human MUC2 gene expression through methylation and the sequence of a translated region adjacent thereto (SEQ ID NO: 8; the DNA sequence after bisulfite treatment, with uracil converted from unmethylated cytosine being indicated as "thymine").
Fig. 3A shows the sequence of a promoter region that regulates human mucin core protein 4 (MUC4) gene expression through methylation and the sequence of a translated region adjacent thereto (SEQ ID NO: 9).
Fig. 3B shows the sequence of a promoter region that regulates human MUC4 gene expression through methylation and the sequence of a translated region adjacent thereto (SEQ ID NO: 10; the DNA sequence after bisulfite treatment, with uracil converted from unmethylated cytosine being indicated as "thymine").
Fig. 4A shows the sequence of a promoter region that regulates human mucin core protein 5AC (MUC5AC) gene expression through methylation (SEQ ID NO: 11).
Fig. 4B shows the sequence of a promoter region that regulates human MUC5AC gene expression through methylation (SEQ ID NO: 12; the DNA sequence after bisulfite treatment, with uracil converted from unmethylated cytosine being indicated as "thymine").
Fig. 5 shows the results of methylation analysis and immunostaining of the IPMN-gastric type.
Fig. 6-1 shows the results of methylation analysis and immunostaining of the IPMN-intestinal type.
Fig. 6-2 shows the results of methylation analysis and immunostaining of the IPMN-intestinal type.
Fig. 7-1 shows the results of methylation analysis and immunostaining of the IPMN-PB type.
Fig. 7-2 shows the results of methylation analysis and immunostaining of the IPMN-PB type.
Fig. 8 shows the results of methylation analysis and immunostaining of PDAC.
Fig. 9 shows the results of statistical analysis based on methylation analysis of the MUC1 gene.
Fig. 10 shows the results of statistical analysis based on methylation analysis of the MUC2 gene.
Fig. 11 shows the results of statistical analysis based on methylation analysis of the MUC4 gene.
Fig. 12 shows the results of statistical analysis based on methylation analysis of the MUC5AC gene.
Fig. 13 shows disease type prediction based on the results of methylation analysis of 4 types of mucin genes.
Fig. 14 shows disease type prediction based on the results of methylation analysis of 4 types of mucin genes.

### Embodiments for Carrying out the Invention

The examination method for determining pancreatic tumor type according to the present disclosure (hereafter, referred to as "the method of the present disclosure") comprises: a first step of detecting the degree of methylation in a 5'-untranslated region or a region comprising a 5'-untranslated region and a translated region of a gene encoding a mucin core protein in a pancreatic fluid sample from a subject such as a patient with pancreatic tumor; and a second step of identifying a pancreatic tumor, using the degree of methylation as an index value, as being of pancreatic tumor type selected from the group consisting of pancreatic ductal adenocarcinoma, an intraductal papillary mucinous neoplasm of the gastric type, an intraductal papillary mucinous neoplasm of the intestinal type, and an intraductal papillary mucinous neoplasm of the pancreatobiliary type. In other words, the method of the present disclosure is a method for diagnosing pancreatic tumor type, an evaluation method for determining pancreatic tumor type, or a method for collecting information for determination of pancreatic tumor type. According to the method of the present disclosure, pancreatic tumor type can be diagnosed at an early stage using a pancreatic fluid sample that can be obtained in a less invasive manner.

Examples of genes encoding mucin core proteins (or mucin genes) include the mucin core protein (MUC) 1 gene, the MUC2 gene, the MUC4 gene, and the MUC5AC gene. In the step of pancreatic tumor identification of the method of the present disclosure (i.e., the second step), in particular, it is preferable to use a set of genes, including the MUC1 gene, the MUC2 gene, the MUC4 gene, and the MUC5AC gene, as the genes encoding mucin core proteins for the purpose of identification of a pancreatic tumor as being of pancreatic tumor type selected from the group consisting of pancreatic ductal adenocarcinoma, an intraductal papillary mucinous neoplasm of the gastric type, an intraductal papillary mucinous neoplasm of the intestinal type, and an intraductal papillary mucinous neoplasm of the pancreatobiliary type with high accuracy.

A 5'-untranslated region of a gene encoding a mucin core protein that is a target sequence (a target region) for the detection of the degree of methylation is an untranslated region (UTR) located in a 5' upstream region of the gene encoding a mucin core protein, and, in particular, it is a region comprising a promoter region of a gene encoding a mucin core protein. Further, a region comprising a 5'-untranslated region and a translated region of a gene encoding a mucin core protein is a region comprising a 5'-untranslated region, such as a promoter region, and a translated region adjacent thereto (i.e., a coding region).

In the method of the present disclosure, at least one (one or a plurality of) CpG site(s) existing in a promoter region of a gene encoding a mucin core protein or a region comprising such promoter region and a translated region can be a target sequence intended for the detection of the degree of methylation. The term "CpG site" refers to a dinucleotide of 5'-cytosine-guanine-3' (5'-CG-3'). When two or more CpG sites are designated as target sequences, each CpG site can be separately designated as a target sequence, or a region comprising two or more CpG sites can be designated as a target sequence.

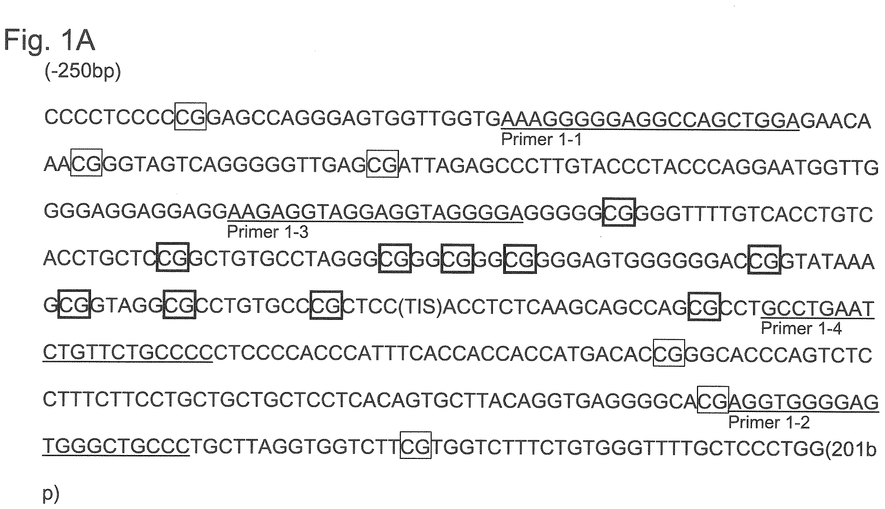

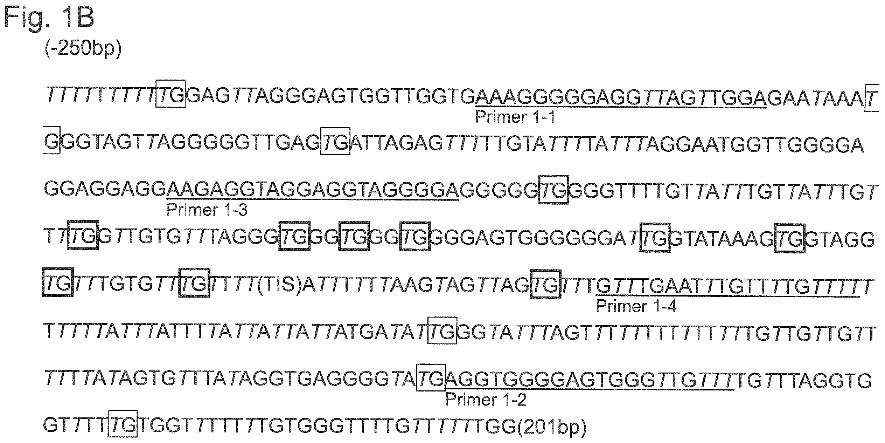

Figs. 1 to 4 each show the sequence of a promoter region that regulates human MUC1, MUC2, MUC4, or MUC5AC gene expression through methylation, and the sequence of a translated region adjacent thereto. In Figs. 1 to 4, "TIS" indicates a transcription initiation site, "CG" enclosed in a square indicates a methylated site (i.e., a CpG site), and "CG" enclosed in a square formed with bold lines indicates a methylated site (i.e., a CpG site) associated with human MUC gene expression. In Fig. 1B, Fig. 2B, Fig. 3B, and Fig. 4B, "T" indicated in italics represents uracil converted from unmethylated cytosine via bisulfite treatment and indicated as "thymine." Nucleotide sequences after the transcription initiation sites (TIS) are translated regions (coding regions).

Fig. 1A shows the sequence of a promoter region that regulates human MUC1 gene expression through methylation and the sequence of a translated region adjacent thereto (SEQ ID NO: 5). Fig. 1B shows the DNA sequence of such sequence after bisulfite treatment (SEQ ID NO: 6), with uracil converted from unmethylated cytosine being indicated as "thymine." In Fig. 1, the region between Primer 1-3 and Primer 1-4 constituting the second set of primers described below (SEQ ID NO: 1) comprises CpG sites (or CpG islands) 172 to 181. As in the case of literature (Norishige Yamada, Yukari Nishida, Hideaki Tsutsumida, Tomofumi Hamada, Masamichi Goto, Michiyo Higashi, Mitsuharu Nomoto, and Suguru Yonezawa, 2008, MUC1 expression is regulated by DNA methylation and histone H3-K9 modification in cancer cells, Cancer Res., 68 (8): 2708-16), CpG sites are numbered successively from the upstream region of the putative promoter region of the human MUC1 gene (2,753 bp upstream from the origin of transcription in the gene).

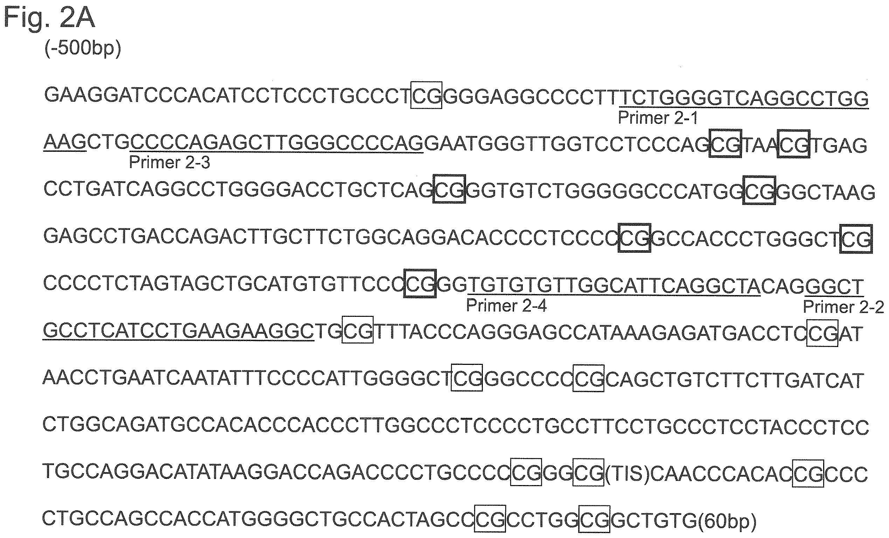

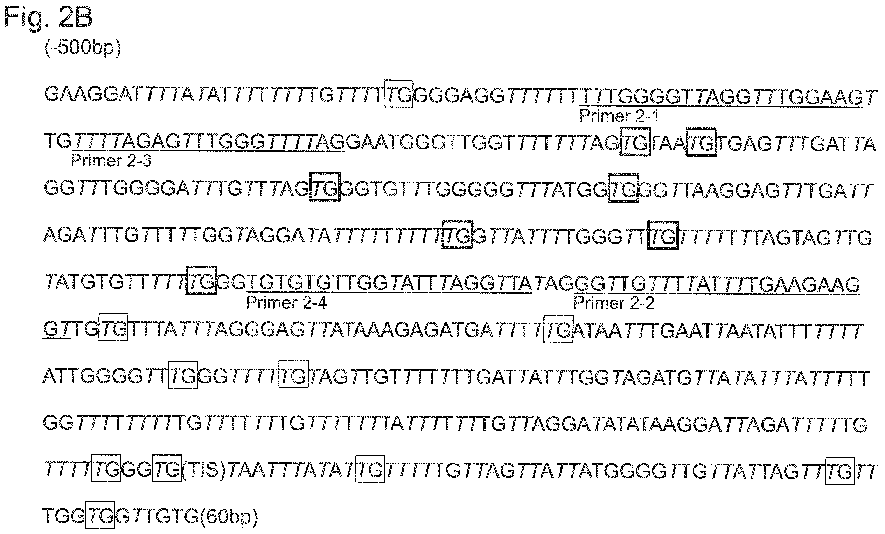

Fig. 2A shows the sequence of a promoter region that regulates human MUC2 gene expression through methylation and the sequence of a translated region adjacent thereto (SEQ ID NO: 7). Fig. 2B shows the DNA sequence of such sequence after bisulfite treatment (SEQ ID NO: 8), with uracil converted from unmethylated cytosine being indicated as "thymine." In Fig. 2, the region between Primer 2-3 and Primer 2-4 constituting the second set of primers described below (SEQ ID NO: 2) comprises CpG sites 37 to 43. As in the case of literature (Norishige Yamada, Tomofumi Hamada, Masamichi Goto, Hideaki Tsutsumida, Michiyo Higashi, Mitsuharu Nomoto, and Suguru Yonezawa, 2006, MUC2 expression is regulated by histone H3 modification and DNA methylation in pancreatic cancer, Int. J. Cancer, 119 (8): 1850-7), CpG sites are numbered successively from the upstream region of the putative promoter region of the human MUC2 gene (1,989 bp upstream from the origin of transcription in the gene).

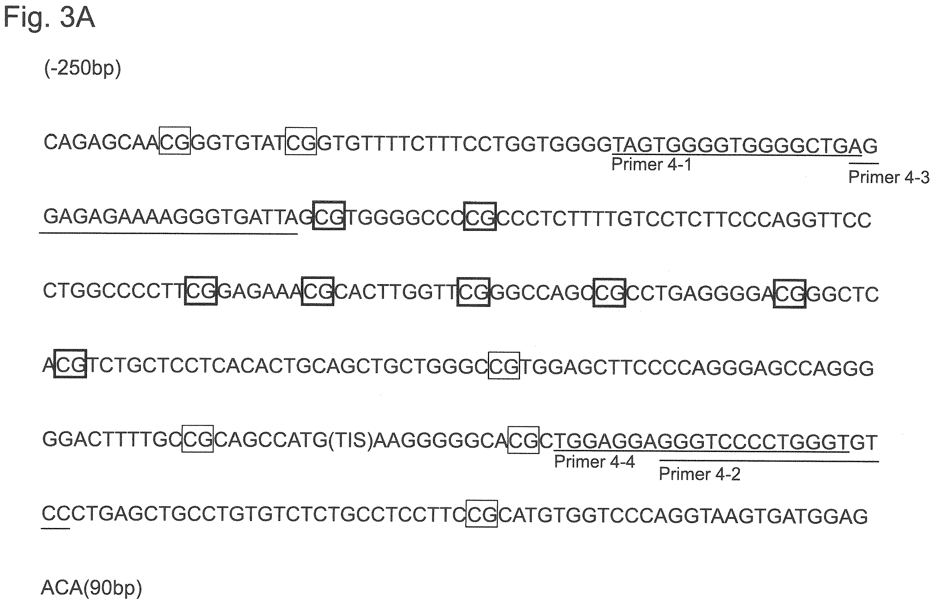

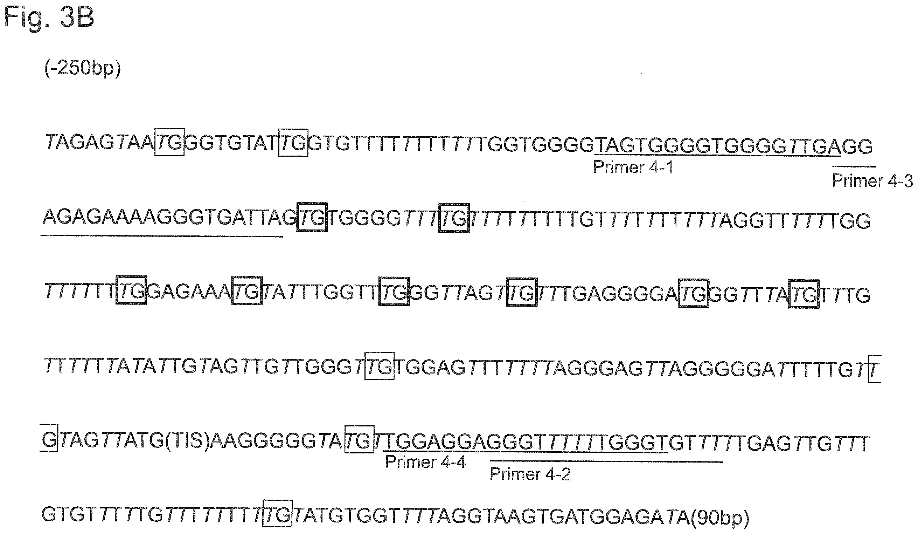

Fig. 3A shows the sequence of a promoter region that regulates human MUC4 gene expression through methylation and the sequence of a translated region adjacent thereto (SEQ ID NO: 9). Fig. 3B shows the DNA sequence of such sequence after bisulfite treatment (SEQ ID NO: 10), with uracil converted from unmethylated cytosine being indicated as "thymine." In Fig. 3, the region between Primer 4-3 and Primer 4-4 constituting the second set of primers described below (SEQ ID NO: 3) comprises CpG sites 108 to 118. As in the case of literature (Norishige Yamada, Yukari Nishida, Hideaki Tsutsumida, Masamichi Goto, Michiyo Higashi, Mitsuharu Nomoto, and Suguru Yonezawa, 2009, Promoter CpG methylation in cancer cells contributes to regulation of MUC4, Br. J. Cancer, 100 (2): 344-51), CpG sites are numbered successively from the upstream region of the putative promoter region of the human MUC4 gene (3,622 bp upstream from the origin of transcription in the gene).

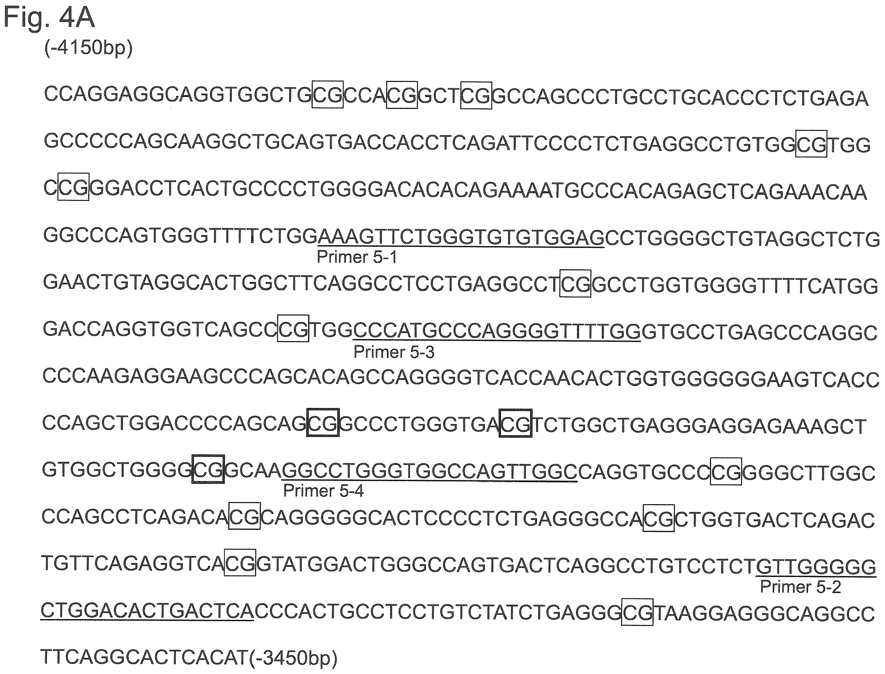

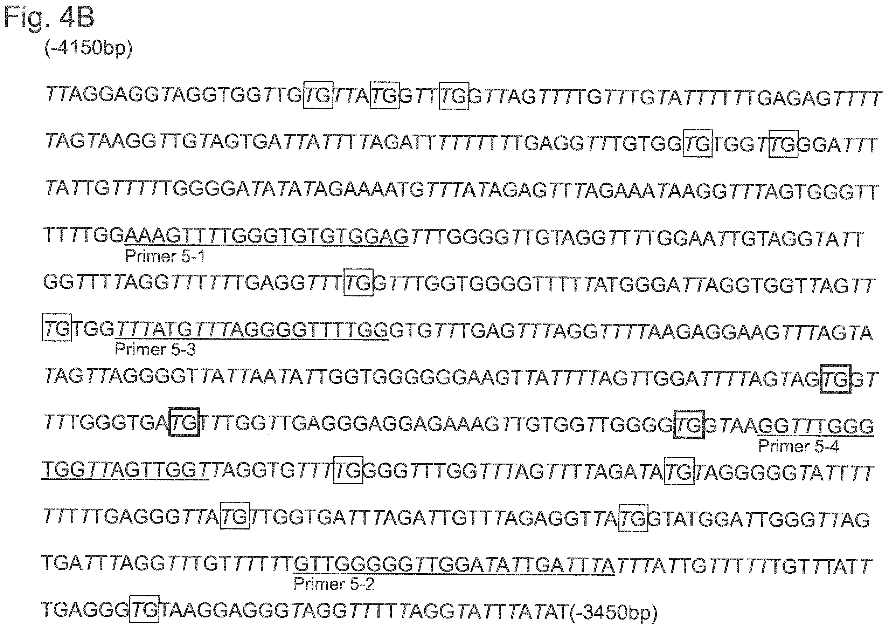

Fig. 4A shows the sequence of a promoter region that regulates human MUC5AC gene expression through methylation (SEQ ID NO: 11). Fig. 4B shows the DNA sequence of such sequence after bisulfite treatment (SEQ ID NO: 12), with uracil converted from unmethylated cytosine being indicated as "thymine." In Fig. 4, the region between Primer 5-3 and Primer 5-4 constituting the second set of primers described below (SEQ ID NO: 4) comprises CpG sites 1 to 3. As in the case of the literature (Yamada N, Nishida Y, Yokoyama S, Tsutsumida H, Houjou I, Kitamoto S, Goto M, Higashi M, and Yonezawa S., Expression of MUC5AC, an early marker of pancreatobiliary cancer, is regulated by DNA methylation in the distal promoter region in cancer cells, J. Hepatobiliary Pancreat. Sci,, 17 (6): 844-854, 2010), CpG sites are numbered successively from the upstream region of the putative promoter region of the human MUC5AC gene (3,718 bp upstream from the origin of transcription in the gene).

In the method of the present disclosure, one or more (one or a plurality of) CpG sites (and, in particular, a methylated region associated with human MUC gene expression) existing in sequences (i.e., the nucleotide sequences as shown in SEQ ID NOs: 1 to 4) between primers of the above second set of primers can be employed as the target sequences subjected to the detection of the degree of methylation as the 5'-untranslated regions or the regions comprising 5'-untranslated regions and translated regions of the human MUC1, MUC2, MUC4, and MUC5AC genes. The nucleotide sequences as shown in SEQ ID NOs: 1 to 4 including all the methylated regions associated with human MUC gene expression (i.e., the CpG sites) or the regions comprising such nucleotide sequences are particularly preferably employed as the target sequences subjected to the detection of the degree of methylation.

Hereafter, the steps of the method of the present disclosure are described. (1) The first step of detecting the degree of methylation in a 5'-untranslated region or a region comprising a 5'-untranslated region and a translated region of a gene encoding a mucin core protein in a pancreatic fluid sample from a subject

In the method of the present disclosure, a pancreatic fluid sample from a subject, such as a patient with pancreatic tumor, is first prepared. For example, a pancreatic fluid sample can be obtained via endoscopic retrograde pancreatography or pancreatic duct mirror examination for diagnostic imaging.

Subsequently, the obtained pancreatic fluid sample is subjected to the detection of the degree of methylation in a 5'-untranslated region or a region comprising a 5'-untranslated region and a translated region of a gene encoding a mucin core protein. The degree of methylation may be detected (or measured) by a known method for detection of methylation. For example, methylated DNA in a particular region may be detected by conducting nucleotide substitution via bisulfite treatment, following which the DNA sequence is then determined. According to such nucleotide substitution, unmethylated cytosine reacts with sodium bisulfite, and it is then converted into uracil. Since methylated cytosine does not react with sodium bisulfite, in principle, the methylated status of all cytosines can be detected as nucleotide differences. Examples of conventional methods for detecting methylation include methylation-specific PCR (MSP) performing PCR, real-time MSP performing quantitative PCR, bisulfite sequencing performing TA cloning, the MassARRAY method performing mass analysis, and Pyrosequencing utilizing a next-generation sequencer. Further, the ICON-prove method, which does not require the bisulfite reaction, has been developed.

The bisulfite-DGGE method was developed by P. Guldberg et al. (Guldberg, P., Gronbak, K., Aggerholm, A., Platz, A., Thor Straten, P., Ahrenkiel, V., Hokland, P., and Zeuthen, J., Detection of mutations in GC-rich DNA by bisulphite denaturing gradient gel electrophoresis, Nucleic Acids Res., 1998, Vol. 26, No. 6, pp. 1548-1549) based on denaturing gradient gel electrophoresis (DGGE) proposed by Abrams and Stanton (Abrams, E. S. and Stanton, V. P. Jr., Use of denaturing gradient gel electrophoresis to study conformational transitions in nucleic acids, Methods Enzymol., 1992, Vol. 212, pp. 71-104). According to this method, the sample after bisulfite treatment is amplified by PCR, and the amplicon thereof is subjected to electrophoresis using a gel consisting of polyacrylamide and a denaturing agent having a density gradient. Based on the density gradient of polyacrylamide, double-stranded DNAs are separated depending on molecular weight. Based on the density gradient of the denaturing agent, further, double-stranded DNAs are separated depending on the degree of denaturation thereof. Double-stranded DNAs are separated based on differences of uracil (unmethylated cytosine) from cytosine (methylated cytosine) in the target region. According to this technique, visual evaluation can be carried out as in the case of the MSP method. In addition, the band observed in a gel after electrophoresis can be subjected to sequencing. In comparison with bisulfite sequencing, the processing time can be shortened to a significant extent with the use of the bisulfide-DGGE method.

In the method of the present disclosure, however, the methylation-specific electrophoresis (MSE) method, which allows detection of DNA methylation in a very small amount of DNA sample and allows detection of the pattern or continuity of DNA methylation, is particularly preferable as a method for detecting the degree of methylation (International Patent Application No. PCT/JP2011/060339 (WO 2011/132798)).

The MSE method comprises steps of: (a) subjecting DNA to bisulfite treatment; (b) subjecting the DNA after bisulfite treatment to a first PCR using a first set of primers corresponding to outer regions of the target region; (c) subjecting the DNA amplified via the first PCR to a second PCR using a second set of primers corresponding to the target region; and (d) subjecting the DNA amplified via the second PCR to denaturing gradient gel electrophoresis.

Hereafter, the steps of the MSE method according to the method of the present disclosure are described.

### (a) Step of subjecting DNA to bisulfite treatment

At the outset, DNA is extracted from a pancreatic fluid sample and then subjected to bisulfite treatment. Thus, unmethylated cytosine is sulfonated with the aid of sodium bisulfite, deaminated via hydrolysis, and then converted into uracil via desulfonation in the presence of alkali. In contrast, methylated cytosine is not converted into uracil via bisulfite treatment. Accordingly, whether or not cytosine in a nucleotide sequence of CpG-containing DNA is methylated is determined based on conversion of cytosine into uracil via bisulfite treatment. DNA can be subjected to bisulfite treatment with the use of a commercially available kit for bisulfite treatment (e.g., an EpiTect Bisulfite Kit, QIAGEN) in accordance with the instructions included therewith.

### (b) Step of subjecting DNA after bisulfite treatment to first PCR

Subsequently, DNA after bisulfite treatment is subjected to a first PCR using a first set of primers corresponding to outer regions of the target region (i.e., a 5'-untranslated region or a region comprising a 5'-untranslated region and a translated region of a gene encoding a mucin core protein). As a result of bisulfite treatment followed by PCR amplification, unmethylated cytosine is converted into thymine, the resulting thymine is mixed with thymine that originally exists in the DNA sequence, and similar sequences thus increase. Such increase leads to misannealing in PCR. When the number of PCR cycles is increased, accordingly, non-specific amplicons are disadvantageously amplified. According to the MSE method, a first PCR is performed using the first set of primers corresponding to outer regions of the target region in order to prevent the occurrence of such noise, and a second PCR (i.e., nested PCR) is then performed to decrease such noise. Thus, the number of PCR cycles can be increased, and the detection limit can be elevated (i.e., trace amounts of DNA samples can be used).

A pair of primers constituting the first set of primers is designed to correspond to outer regions of the target region and anneal to the outer regions. Such outer regions can be located, for example, 10 to 100 nucleotides (preferably 20 to 80 nucleotides) away from each end of the target region in outward directions. The first set of primers can be composed of, for example, 15 to 30 nucleotides, and preferably 18 to 22 nucleotides.

PCR can be carried out by, for example, taking the length of the amplification product or GC content into consideration and adequately determining the composition of a PCR reaction solution (e.g., PCR buffer, polymerase dNTP mix, or primers), the temperature conditions, and the number of cycles for thermal denaturation, annealing, and extension. Such PCR conditions can be adequately determined (e.g., optimal conditions for polymerase to be used). Uracil converted from unmethylated cytosine is converted into thymine via such PCR.

### (c) Step of subjecting DNA amplified via first PCR to second PCR

After the first PCR, DNA amplified via the first PCR (i.e., an amplification product) is subjected to a second PCR using a second set of primers corresponding to the target region. The second PCR is referred to as "nested PCR." Specifically, the second set of primers are located within the amplification product after the first PCR, and a second PCR is carried out using the amplification product after the first PCR as a new template.

The second set of primers are designed in such a manner that the annealing positions thereof are located inside the annealing positions of the first set of primers relative to the target region. Specifically, the annealing positions of the second set of primers are at both ends of the target region or regions adjacent thereto. The second set of primers may partially overwrap the first set of primers, provided that the annealing positions of the second set of primers are located inside the annealing positions of the first set of primers. The second set of primers can comprise, for example, 15 to 30 nucleotides, and preferably 18 to 22 nucleotides. By adding a GC-clamp sequence to the 5' side of one of a pair of primers constituting the second set of primers (e.g., a forward (sense) primer), the separability can be improved in the subsequent step of denaturing gradient gel electrophoresis. The term "GC-clamp sequence" used herein refers to a G-C-rich stable sequence comprising about 30 to 50 nucleotides. An example thereof is the nucleotide sequence shown in SEQ ID NO: 13.

As with the case of the first PCR, PCR can be carried out by, for example, taking the length of the target region or GC content into consideration and adequately determining the composition of a PCR reaction solution (e.g., PCR buffer, polymerase dNTP mix, or primers), as well as the temperature conditions and the number of cycles for thermal denaturation, annealing, and extension.

### (d) Step of subjecting DNA amplified via second PCR to denaturing gradient gel electrophoresis

After the second PCR, amplified DNA can be subjected to denaturing gradient gel electrophoresis, so that the amplified DNA can be separated, and a pattern or continuity of methylation can be visually evaluated.

A denaturing gradient gel is prepared using, for example, a gel component (acrylamide), a denaturing agent, such as a combination of urea and formamide, and TAE buffer. When a polyacrylamide gel is used and a combination of urea and formamide is used as a denaturing agent, specifically, a denaturing gradient gel is prepared so as to bring the acrylamide density to, for example, 6% to 15% (preferably 8% to 10%) and the density gradient of the combination of urea and formamide to, for example, 10% → 50% to 20% → 30% (with density gradient interval preferably being 10% or higher), in accordance with the length of the amplification product or other conditions. Optimal conditions are examined with reference to a gel with a broad density gradient interval (10% to 50%), and the method for narrowing the density gradient interval is examined. When the detected band exhibits a smear, acrylamide density is increased. The gradient of the denaturing agent in the gel is adjusted to increase in the direction from the cathode to the anode of electrophoresis (i.e., the direction of DNA migration).

After the second PCR, the amplified DNA (e.g., 4 to 15 µl, and preferably 5 to 10 µl) is applied to a denaturing gradient gel and then subjected to electrophoresis. Electrophoresis is carried out in an electrophoresis bath at a temperature of 60°C and a constant voltage of 70 to 250 V for 300 to 900 minutes, for example.

After electrophoresis, the denaturing gradient gel is subjected to ethidium bromide staining or GelRed staining, so that the band of the applied amplified DNA can be visually observed. A GC pair is bound by three hydrogen bonds, and an AT pair is bound by two hydrogen bonds. Accordingly, a GC bond is more tolerant to a denaturing agent than an AT bond. Thus, the migration level of DNA having many GC bonds in a denaturing gradient gel is higher than that of DNA having many AT bonds.

Since unmethylated cytosine in DNA is converted into uracil and then into thymine, the velocity of migration of amplified DNA corresponding to a target region having unmethylated cytosine is slower than that of amplified DNA corresponding to a target region having methylated cytosine, and DNA of the former type is located at a position closer to the cathode of the denaturing gradient gel. In contrast, the velocity of migration of amplified DNA corresponding to the target region having methylated cytosine is fast, and such DNA is located at a position closer to the anode of the denaturing gradient gel. Accordingly, a target region having methylated cytosine can be distinguished from a target region having unmethylated cytosine based on such differences in migration behavior, and a pattern or continuity of methylation in the target region can be evaluated.
(2) Second step of identifying pancreatic tumor as being of pancreatic tumor type selected from the group consisting of pancreatic ductal adenocarcinoma, intraductal papillary mucinous neoplasm of the gastric type, intraductal papillary mucinous neoplasm of the intestinal type, and intraductal papillary mucinous neoplasm of the pancreatobiliary type using the detected degrees of methylation as index values

In the method of the present disclosure, a pancreatic tumor is identified as pancreatic ductal adenocarcinoma (PDAC), an intraductal papillary mucinous neoplasm of the gastric type (IPMN-gastric), an intraductal papillary mucinous neoplasm of the intestinal type (IPMN-intestinal), or an intraductal papillary mucinous neoplasm of the pancreatobiliary type (IPMN-PB) using the detected degree of methylation as an index value.

For example, the emission intensity of a band in a photograph showing a gel after DGGE in the MSE method is quantified using software for image processing, such as Image J, and subjected to statistical processing using software for statistical analysis (e.g., R, software for statistical analysis). Methylated DNA contents determined by such statistical analysis are compared. When a band indicating an unmethylated status is observed but a high content of methylated DNA is observed as a whole, for example, the gene of interest is evaluated as being methylated. The methylated or unmethylated statuses of genes in a set including, for example, the MUC1 gene, the MUC2 gene, the MUC4 gene, and the MUC5AC gene are evaluated by designating cut-off values for methylated DNA content as 50% (±20%) for the MUC1 gene, 70% (±20%) for the MUC2 gene, 50% (±20%) for the MUC4 gene, and 60% (±20%) for the MUC5AC gene. Disease type prediction is then performed using such 4 types of genes encoding mucin core proteins to identify the pancreatic tumor as: (a) IPMN-gastric if all 4 genes are methylated; (b) IPMN-intestinal if all 4 genes are unmethylated; (c) PDAC if the MUC1, MUC2, and MUC4 genes are unmethylated and the MUC5AC gene is methylated; or (d) IPMN-PB if none of the above is applicable. Early diagnosis can be performed, including evaluation as to whether a detected tumor is PDAC with a very poor prognosis or an intraductal papillary mucinous tumor (IPMN) with a relatively good prognosis, or IPMN-intestinal with a high risk of developing cancer or IPMN-gastric with relative safety among IPMNs.

The method of the present disclosure described above is a diagnostic method involving the use of a pancreatic fluid sample that can be obtained in a less invasive manner. Such diagnosis can be carried out in addition to various currently available testing techniques (e.g., histological diagnosis), so that pancreatic tumors can be more accurately diagnosed. According to the method of the present disclosure, detection of an epigenetic anomaly is performed with the effective use of an excreted fluid; i.e., a pancreatic fluid, which can be obtained in a less invasive manner, in addition to conventional image diagnostic techniques that have been developed to a high degree. Thus, a clear therapeutic regimen can be established at an early stage based on qualitative diagnosis that assesses malignancy.

Also, the present disclosure relates to an examination (test) kit for determination of pancreatic tumor type, which is used for implementing the method of the present disclosure (hereafter, merely referred to as a "kit"). Such kit can be a kit for diagnosis of pancreatic tumor type, an evaluation kit for determination of pancreatic tumor type, or a kit for collecting information for determination of pancreatic tumor type.

A kit can contain, for example, a reagent used for carrying out the MSE method. Examples of reagents include bisulfite (sodium bisulfite) used for bisulfite treatment; the first set of primers used for the first PCR; the second set of primers used for the second PCR; components of reaction solutions for the first PCR and the second PCR (e.g., PCR buffer, polymerase, and dNTP mix); and a gel component (e.g., acrylamide), a denaturing agent (e.g., urea or formamide) and buffer (e.g., TAE buffer) used for denaturing gradient gel electrophoresis.

As described in the examples below, examples of the first set of primers include: a set of a primer comprising the nucleotide sequence as shown in SEQ ID NO: 14 and a primer comprising the nucleotide sequence as shown in SEQ ID NO: 15 for the MUC1 gene; a set of a primer comprising the nucleotide sequence as shown in SEQ ID NO: 18 and a primer comprising the nucleotide sequence as shown in SEQ ID NO: 19 for the MUC2 gene; a set of a primer comprising the nucleotide sequence as shown in SEQ ID NO: 22 and a primer comprising the nucleotide sequence as shown in SEQ ID NO: 23 for the MUC4 gene; and a set of a primer comprising the nucleotide sequence as shown in SEQ ID NO: 26 and a primer comprising the nucleotide sequence as shown in SEQ ID NO: 27 for the MUC5AC gene. Examples of the second set of primers include: a set of a primer comprising the nucleotide sequence as shown in SEQ ID NO: 16 and a primer comprising the nucleotide sequence as shown in SEQ ID NO: 17 for the MUC1 gene; a set of a primer comprising the nucleotide sequence as shown in SEQ ID NO: 20 and a primer comprising the nucleotide sequence as shown in SEQ ID NO: 21 for the MUC2 gene; a set of a primer comprising the nucleotide sequence as shown in SEQ ID NO: 24 and a primer comprising the nucleotide sequence as shown in SEQ ID NO: 25 for the MUC4 gene; and a set of a primer comprising the nucleotide sequence as shown in SEQ ID NO: 28 and a primer comprising the nucleotide sequence as shown in SEQ ID NO: 29 for the MUC5AC gene.

In addition, the kit can comprise the instructions and the operating manuals for determination of pancreatic tumor type, and the like.

Hereafter, the present invention is described in greater detail with reference to the following examples.

### [Example 1] Pancreatic tumor type prediction through methylation analysis of 4 types of mucin genes

In Example 1, methylation analysis of the methylated sites (CpG sites) associated with the expression on the MUC1, MUC2, MUC4, and MUC5AC gene promoter regions or translated regions adjacent thereto is carried out by the MSE method using pancreatic fluid samples of human pancreatic ductal adenocarcinoma (PDAC), human intraductal papillary mucinous neoplasm of the gastric type (IPMN-gastric), human intraductal papillary mucinous neoplasm of the intestinal type (IPMN-intestinal), and human intraductal papillary mucinous neoplasm of the pancreatobiliary type (IPMN-PB). In addition, expression of proteins encoded by such genes was examined via immunohistochemical staining.

### 1. Materials and Methods

### 1-1. Samples

The samples subjected to analysis are shown in Table 1.

**Table 1**

| | |
|---|---|
| IPMN-gastric | 6 samples |
| IPMN-intestinal | 6 samples |
| IPMN-PB | 4 samples |
| PDAC | 2 samples |
| Total | 18 samples |

### 1-2. Methylation analysis of methylated site (CpG site) associated with MUC1 gene promoter expression via MSE

A methylated site (a CpG site) associated with human MUC1 gene promoter expression was analyzed by the MSE method. Fig. 1A shows the sequence of a promoter region that regulates human MUC1 gene expression through methylation and the sequence of a translated region adjacent thereto (SEQ ID NO: 5). Fig. 1B shows the DNA sequence of such sequence after bisulfite treatment (SEQ ID NO: 6), with uracil converted from unmethylated cytosine being indicated as "thymine." In Fig. 1, the region between Primer 1-3 and Primer 1-4 described below (SEQ ID NO: 1) comprises CpG sites 172 to 181, and a region comprising these CpG sites is designated as the target region. As in the case of literature (Norishige Yamada, Yukari Nishida, Hideaki Tsutsumida, Tomofumi Hamada, Masamichi Goto, Michiyo Higashi, Mitsuharu Nomoto, and Suguru Yonezawa, 2008, MUC1 expression is regulated by DNA methylation and histone H3-K9 modification in cancer cells, Cancer Res., 68 (8): 2708-16), CpG sites are numbered successively from the upstream region of the putative promoter region of the human MUC1 gene (2,753 bp upstream from the origin of transcription in the gene).

DNA was extracted from a pancreatic fluid sample using the DNeasy Blood & Tissue Kit (QIAGEN).

Subsequently, the extracted DNA was subjected to bisulfite treatment using the EpiTect Bisulfite Kit (QIAGEN).

The DNA samples after bisulfite treatment were subjected to PCR performed with the use of the primers below.

Set of primers (the nucleotide sequence indicated by lower-case letters is the GC clamp)
Primer 1-1: 5'-AAAGGGGGAGGTTAGTTGGA-3' (SEQ ID NO: 14)
Primer 1-2: 5'-AAACAACCCACTCCCCACCT-3' (SEQ ID NO: 15)
Primer 1-3:
   5'-cgcccgccgcgcgcggcgggcggggcgggggcacggggggAAGAGGTAGGAGGTAGGGGA-3' (SEQ ID NO: 16)
Primer 1-4: 5'-AAAACAAAACAAATTCAAAC-3' (SEQ ID NO: 17)

As shown in Fig. 1, 1^{st} PCR was carried out using Primer 1-1 and Primer 1-2, and 2^{nd} PCR (nested PCR) was carried out using Primer 1-3 and Primer 1-4. AmpliTaq Gold® Fast PCR Master Mix (Applied Biosystem) was used as polymerase. PCR conditions and temperature conditions are shown in Table 2 below.

**Table 2**

| Composition of reaction solution for 1^{st} PCR | | | Composition of reaction solution for 2^{nd} PCR | | |
|---|---|---|---|---|---|
| DNA after bisulfite treatment | | 1µl | 1^{st} PCR amplicon | | 2µl |
| Master Mix | | 10µl | Master Mix | | 1µl |
| Primer 1-1 | | 0.3µl | Primer 1-3 | | 0.3µl |
| Primer 1-2 | | 0.3µl | Primer 1-4 | | 0.3µl |
| dH₂O | | 8.4µl | dH₂O | | 7.4µl |
| Total | | 20µl | Total | | 20µl |
| | | | | | |

| 1^{st} PCR temperature conditions | | | 2^{nd} PCR temperature conditions | | |
|---|---|---|---|---|---|
| 95°C 10 min | | | 95°C 10 min | | |
| 96°C 5 sec | } 40 cycles | | 96°C 5 sec | } 45 cycles | |
| 63°C 5 sec | | | 53°C 5 sec | | |
| 68°C 9 sec | | | 68°C 9 sec | | |
| 72°C 10 sec | | | 72°C 10 sec | | |

Subsequently, the reaction solution after the 2^{nd} PCR was subjected to DGGE using a denaturing gradient gel having the conditions for DGGE gel shown in Table 3 below. Electrophoresis was carried out in an electrophoresis bath at a temperature of 60°C and a constant voltage of 230 V for 300 minutes. The DCode System (BIO-RAD) was employed as an electrophoresis bath.

**Table 3**

| Conditions for DGGE gel | | | |
|---|---|---|---|
| 10% acrylamide gel | | | |
| Denaturing density gradient: 30% to 40% | | | |
| | | | |

| Composition of 30% denaturing gel | | Composition of 40% denaturing gel | |
|---|---|---|---|
| 40% stock solution | 3.0ml | 40% stock solution | 3.0ml |
| 50×TAE buffer | 0.3ml | 50xTAE buffer | 0.3ml |
| Urea | 1.88g | Urea | 2.51 g |
| Formamide | 1.8ml | Formamide | 2.4ml |

40% stock solution: BIO-RAD, 40(w/v)%-Acrylamide/Bis Mixed Solution(37.5:1);
50X TAE buffer: nacalai tesque, Tris-Acetate-EDTA Buffer(50x);
Urea: nacalai tesque, SP grade;
Formamide: nacalai tesque, SP grade

### 1-3. Methylation analysis of methylated site (CpG site) associated with MUC2 gene promoter expression via MSE

A methylated site (a CpG site) associated with human MUC2 gene promoter expression was analyzed by the MSE method. Fig. 2A shows the sequence of a promoter region that regulates human MUC2 gene expression through methylation and the sequence of a translated region adjacent thereto (SEQ ID NO: 7). Fig. 2B shows the DNA sequence of such sequence after bisulfite treatment (SEQ ID NO: 8), with uracil converted from unmethylated cytosine being indicated as "thymine." In Fig. 2, the region between Primer 2-3 and Primer 2-4 described below (SEQ ID NO: 2) comprises CpG sites 37 to 43, and a region comprising these CpG sites is designated as the target region. As in the case of literature (Norishige Yamada, Tomofumi Hamada, Masamichi Goto, Hideaki Tsutsumida, Michiyo Higashi, Mitsuharu Nomoto, and Suguru Yonezawa, 2006, MUC2 expression is regulated by histone H3 modification and DNA methylation in pancreatic cancer, Int. J. Cancer, 119 (8): 1850-7), CpG sites are numbered successively from the upstream region of the putative promoter region of the human MUC2 gene (1,989 bp upstream from the origin of transcription in the gene).

In the same manner as in Section 1-2 above, DNA was extracted from a pancreatic fluid sample using the DNeasy Blood & Tissue Kit (QIAGEN), and the extracted DNA was subjected to bisulfite treatment using the EpiTect Bisulfite Kit (QIAGEN).

The DNA samples after bisulfite treatment were subjected to PCR performed with the use of the primers below.
Set of primers (the nucleotide sequence indicated by lower-case letters is the GC clamp)
Primer 2-1: 5'-TTTGGGGTTAGGTTTGGAAG-3' (SEQ ID NO: 18)
Primer 2-2: 5'-ACCTTCTTCAAAATAAAACAACC-3' (SEQ ID NO: 19)
Primer 2-3: 5'-cgcccgccgcgcgcggcgggcggggcgggggcacggggggTTTTAGAGTTTGGGTTTTAG-3' (SEQ ID NO: 20)
Primer 2-4: 5'-TAACCTAAATACCAACACACA-3' (SEQ ID NO: 21)

As shown in Fig. 2, 1^{st} PCR was carried out using Primer 2-1 and Primer 2-2, and 2^{nd} PCR (nested PCR) was carried out using Primer 2-3 and Primer 2-4. AmpliTaq Gold® Fast PCR Master Mix (Applied Biosystem) was used as polymerase. PCR conditions and temperature conditions are shown in Table 4 below.

**Table 4**

| Composition of reaction solution for 1^{st} PCR | | | Composition of reaction solution for 2^{nd} PCR | | |
|---|---|---|---|---|---|
| DNA after bisulfite treatment | | 1µl | 1^{st} PCR amplicon | | 2µl |
| Master Mix | | 10µl | Master Mix | | 10µl |
| Primer 2-1 | | 0.3µl | Primer 2-3 | | 0.3µl |
| Primer 2-2 | | 0.3µl | Primer 2-4 | | 0.3µl |
| dH₂O | | 8.4µl | dH₂O | | 7.4µl |
| Total | | 20µl | Total | | 20µl |
| | | | | | |

| 1^{st} PCR temperature conditions | | | 2^{nd} PCR temperature conditions | | |
|---|---|---|---|---|---|
| 95°C 10 min | | | 95°C 10 min | | |
| 96°C 5 sec | } 40 cycles | | 96°C 5 sec | } 45 cycles | |
| 59°C 5 sec | | | 51 °C 5 sec | | |
| 68°C 9 sec | | | 68°C 9 sec | | |
| 72°C 10 sec | | | 72°C 10 sec | | |

Subsequently, the reaction solution after the 2^{nd} PCR was subjected to DGGE using a denaturing gradient gel having the conditions for DGGE gel shown in Table 5 below. The electrophoresis conditions and the electrophoresis bath employed in Section 1-2 above were also employed herein.

**Table 5**

| Conditions for DGGE gel | | | |
|---|---|---|---|
| 10% acrylamide gel | | | |
| Denaturing density gradient: 20% to 28% | | | |
| | | | |

| Composition of 20% denaturing gel | | Composition of 28% denaturing gel | |
|---|---|---|---|
| 40% stock solution | 3.0ml | 40% stock solution | 3.0ml |
| 50×TAE buffer | 0.3ml | 50×TAE buffer | 0.3ml |
| Urea | 1.25g | Urea | 1.76g |
| Formamide | 1.2ml | Formamide | 1.7ml |

40% stock solution: BIO-RAD, 40(w/v)%-Acrylamide/Bis Mixed Solution(37.5:1); 50X TAE buffer: nacalai tesque, Tris-Acetate-EDTA Buffer(50x);
Urea: nacalai tesque, SP grade;
Formamide: nacalai tesque, SP grade

### 1-4. Methylation analysis of methylated site (CpG site) associated with MUC4 gene promoter expression via MSE

A methylated site (a CpG site) associated with human MUC4 gene promoter expression was analyzed by the MSE method. Fig. 3A shows the sequence of a promoter region that regulates human MUC4 gene expression through methylation and the sequence of a translated region adjacent thereto (SEQ ID NO: 9). Fig. 3B shows the DNA sequence of such sequence after bisulfite treatment (SEQ ID NO: 10), with uracil converted from unmethylated cytosine being indicated as "thymine." In Fig. 3, the region between Primer 4-3 and Primer 4-4 described below (SEQ ID NO: 3) comprises CpG sites 108 to 118, and a region comprising these CpG sites is designated as the target region. As in the case of literature (Norishige Yamada, Yukari Nishida, Hideaki Tsutsumida, Masamichi Goto, Michiyo Higashi, Mitsuharu Nomoto, and Suguru Yonezawa, 2009, Promoter CpG methylation in cancer cells contributes to regulation of MUC4, Br. J. Cancer, 100 (2): 344-51), CpG sites are numbered successively from the upstream region of the putative promoter region of the human MUC4 gene (3,622 bp upstream from the origin of transcription in the gene).

In the same manner as in Section 1-2 above, DNA was extracted from a pancreatic fluid sample using the DNeasy Blood & Tissue Kit (QIAGEN), and the extracted DNA was subjected to bisulfite treatment using the EpiTect Bisulfite Kit (QIAGEN).

The DNA samples after bisulfite treatment were subjected to PCR performed with the use of the primers below.
Set of primers (the nucleotide sequence indicated by lower-case letters is the GC clamp)
Primer 4-1: 5'-TAGTGGGGTGGGGTTGA-3' (SEQ ID NO: 22)
Primer 4-2: 5'-AAACACCCAAAAAACCC-3' (SEQ ID NO: 23)
Primer 4-3: 5'-cgcccgccgcgcgcggcgggcggggcgggggcacggggggAGGAGAGAAAAGGGTGATTA-3' (SEQ ID NO: 24)
Primer 4-4: 5'-ACCCAAAAAACCCTCCTCCA-3' (SEQ ID NO: 25)

As shown in Fig. 3, 1^{st} PCR was carried out using Primer 4-1 and Primer 4-2, and 2^{nd} PCR (nested PCR) was carried out using Primer 4-3 and Primer 4-4. AmpliTaq Gold® Fast PCR Master Mix (Applied Biosystem) was used as polymerase. PCR conditions and temperature conditions are shown in Table 6 below.

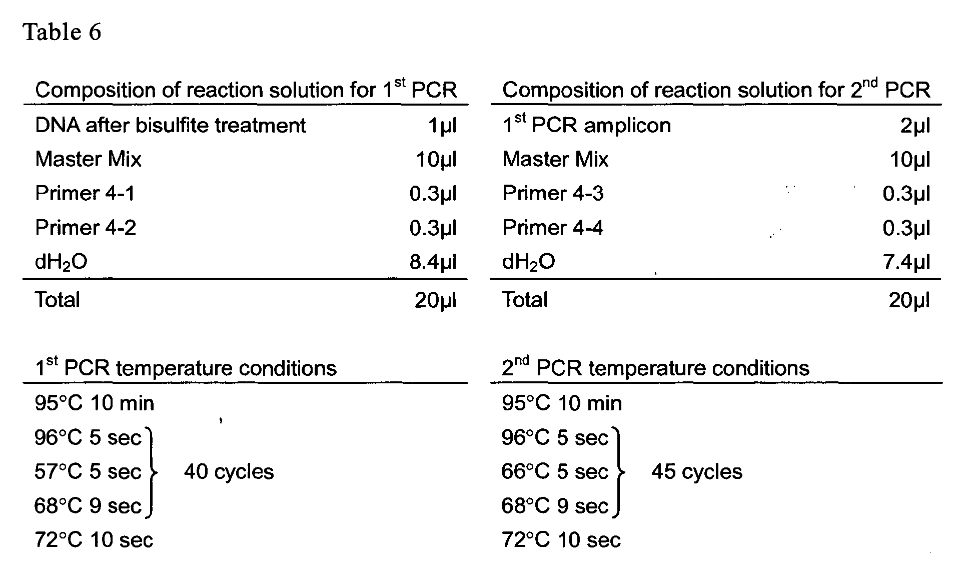

Subsequently, the reaction solution after the 2^{nd} PCR was subjected to DGGE using a denaturing gradient gel having the conditions for DGGE gel shown in Table 7 below. The electrophoresis conditions and the electrophoresis bath employed in Section 1-2 above were also employed herein.

**Table 7**

| Conditions for DGGE gel | | | |
|---|---|---|---|
| 10% acrylamide gel | | | |
| Denaturing density gradient: 25% to 45% | | | |
| | | | |

| Composition of 25% denaturing gel | | Composition of 45% denaturing gel | |
|---|---|---|---|
| 40% stock solution | 3.0ml | 40% stock solution | 3.0ml |
| 50×TAE buffer | 0.3ml | 50×TAE buffer | 0.3ml |
| Urea | 1.57g | Urea | 2.82g |
| Formamide | 1.5ml | Formamide | 2.7ml |

| | | | |
|---|---|---|---|
| 40% stock solution: BIO-RAD, 40(w/v)%-Acrylamide/Bis Mixed Solution(37.5:1); 50X TAE buffer: nacalai tesque, Tris-Acetate-EDTA Buffer(50x); Urea: nacalai tesque, SP grade; Formamide: nacalai tesque, SP grade | | | |

### 1-5. Methylation analysis of methylated site (CpG site) associated with MUC5AC gene promoter expression via MSE

A methylated site (a CpG site) associated with human MUC5AC gene promoter expression was analyzed by the MSE method. Fig. 4A shows the sequence of a promoter region that regulates human MUC5AC gene expression through methylation (SEQ ID NO: 11). Fig. 4B shows the DNA sequence of such sequence after bisulfite treatment (SEQ ID NO: 12), with uracil converted from unmethylated cytosine being indicated as "thymine." In Fig. 4, the region between Primer 5-3 and Primer 5-4 described below (SEQ ID NO: 4) comprises CpG sites 1 to 3, and a region comprising these CpG sites is designated as the target region. As in the case of literature (Yamada N, Nishida Y, Yokoyama S, Tsutsumida H, Houjou I, Kitamoto S, Goto M, Higashi M, and Yonezawa S., 2010, Expression of MUC5AC, an early marker of pancreatobiliary cancer, is regulated by DNA methylation in the distal promoter region in cancer cells, J. Hepatobiliary Pancreat. Sci., 17 (6): 844-854), CpG sites are numbered successively from the upstream region of the putative promoter region of the human MUC5AC gene (3,718 bp upstream from the origin of transcription in the gene).

In the same manner as in Section 1-2 above, DNA was extracted from a pancreatic fluid sample using the DNeasy Blood & Tissue Kit (QIAGEN), and the extracted DNA was subjected to bisulfite treatment using the EpiTect Bisulfite Kit (QIAGEN).

The DNA samples after bisulfite treatment were subjected to PCR performed with the use of the primers below.
Set of primers (the nucleotide sequence indicated by lower-case letters is the GC clamp)
Primer 5-1: 5'-AAAGTTTTGGGTGTGTGGAG-3' (SEQ ID NO: 26)
Primer 5-2: 5'-TAAATCAATATCCAACCCCCAAC-3' (SEQ ID NO: 27)
Primer 5-3: 5'-cgcccgccgcgcgcggcgggcggggcgggggcacggggggTTTATGTTTAGGGGTTTTGG-3' (SEQ ID NO: 28)
Primer 5-4: 5'-ACCAACTAACCACCCAAACC-3' (SEQ ID NO: 29)

As shown in Fig. 4, 1^{st} PCR was carried out using Primer 5-1 and Primer 5-2, and 2^{nd} PCR (nested PCR) was carried out using Primer 5-3 and Primer 5-4. AmpliTaq Gold® Fast PCR Master Mix (Applied Biosystem) was used as polymerase. PCR conditions and temperature conditions are shown in Table 8 below.

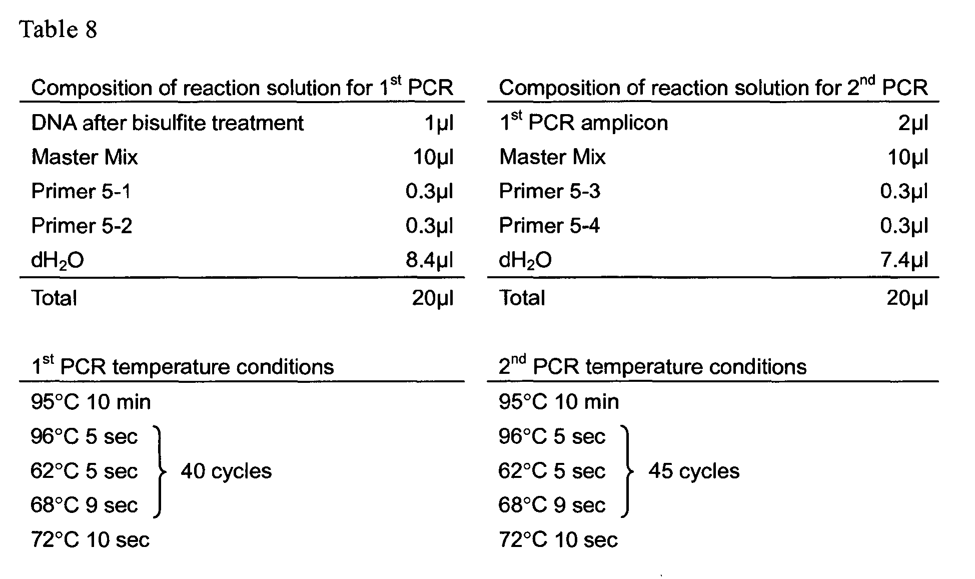

Subsequently, the reaction solution after the 2^{nd} PCR was subjected to DGGE using a denaturing gradient gel having the conditions for DGGE gel shown in Table 9 below. Electrophoresis was carried out in an electrophoresis bath at a temperature of 60°C and a constant voltage of 230 V for 300 minutes. The DCode System (BIO-RAD) was employed as an electrophoresis bath.

**Table 9**

| Conditions for DGGE gel | | | |
|---|---|---|---|
| 10% acrylamide gel | | | |
| Denaturing density gradient: 30% to 40% | | | |
| | | | |

| Composition of 30% denaturing gel | | Composition of 40% denaturing gel | |
|---|---|---|---|
| 40% stock solution | 3.0ml | 40% stock solution | 3.0ml |
| 50×TAE buffer | 0.3ml | 50×TAE buffer | 0.3ml |
| Urea | 1.88g | Urea | 2.51g |
| Formamide | 1.8ml | Formamide | 2.4ml |

| | | | |
|---|---|---|---|
| 40% stock solution: BIO-RAD, 40(w/v)%-Acrylamide/Bis Mixed Solution(37.5:1); 50X TAE buffer: nacalai tesque, Tris-Acetate-EDTA Buffer(50x); Urea: nacalai tesque, SP grade; Formamide: nacalai tesque, SP grade | | | |

### 1-6. Expression analysis of protein encoded by MUC gene via immunohistochemical staining

The human PDAC, IPMN-gastric, IPMN-intestinal, and IPMN-PB tissue samples obtained via surgery were subjected to expression analysis of proteins encoded by the MUC1, MUC2, MUC4, and MUC5AC genes via immunohistochemical staining.

When immunohistochemical staining was performed for the purpose of protein expression analysis, a novel anti-MUC1 antibody against the MUC1 cytoplasmic tail domain (derived from the hybridoma strain (i.e., the MUC1-common clone 014E); JP Patent Application No. 2010-097922 (JP 2011-184427 A); Accession Number: NITE BP-867) was used for MUC1 analysis, in addition to a conventional anti-MUC1 antibody (MUC1-DF3; TFB). MUC2 was analyzed with the use of the anti-MUC2 antibody (MUC2-Ccp58; Novo). MUC4 was analyzed with the use of the anti-MUC4 antibody (MUC4-8G7) against the N-terminal subunit (University of Nebraska Medical Center, Omaha) and the anti-MUC4 antibody (MUC4-1G8) against the C-terminal subunit (Zymed). MUC5AC was analyzed with the use of the anti-MUC5AC antibody (Muc5AC, Novo).

### 2. Results

### 2-1. Results of methylation analysis and immunostaining

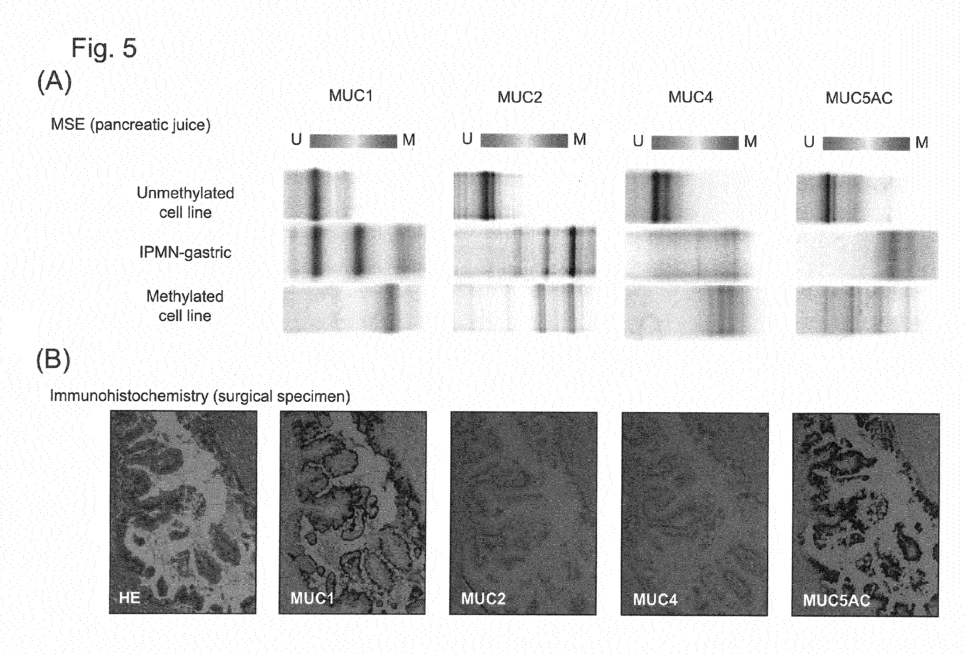

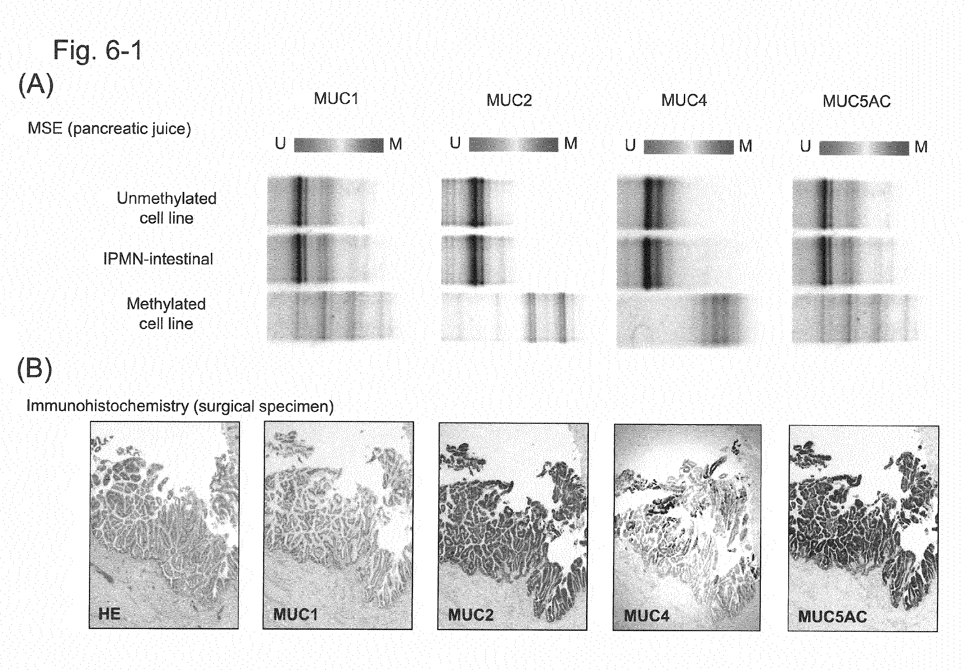

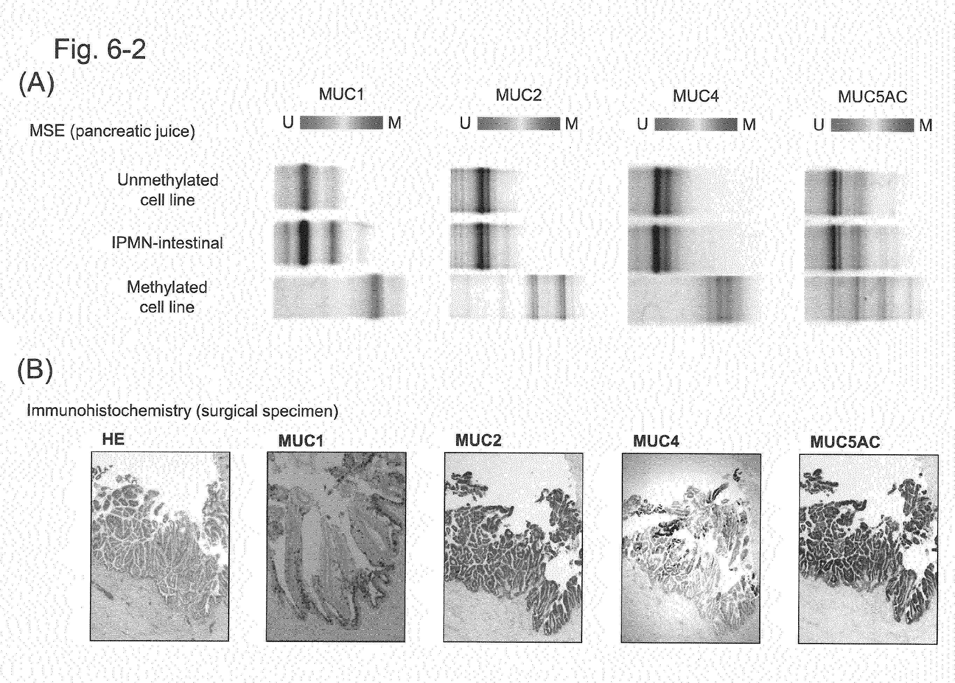

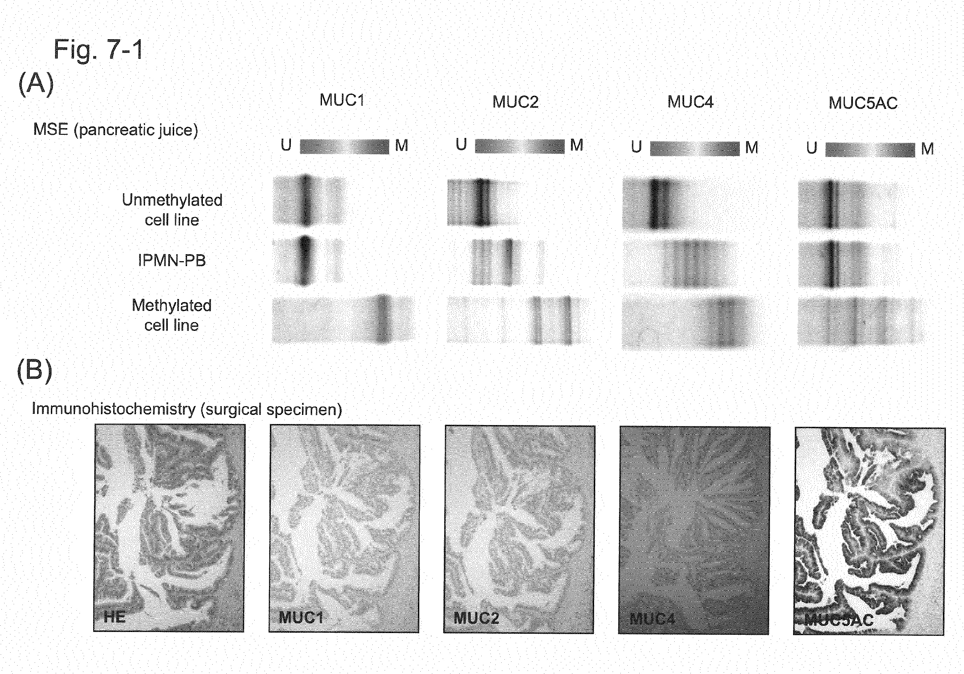

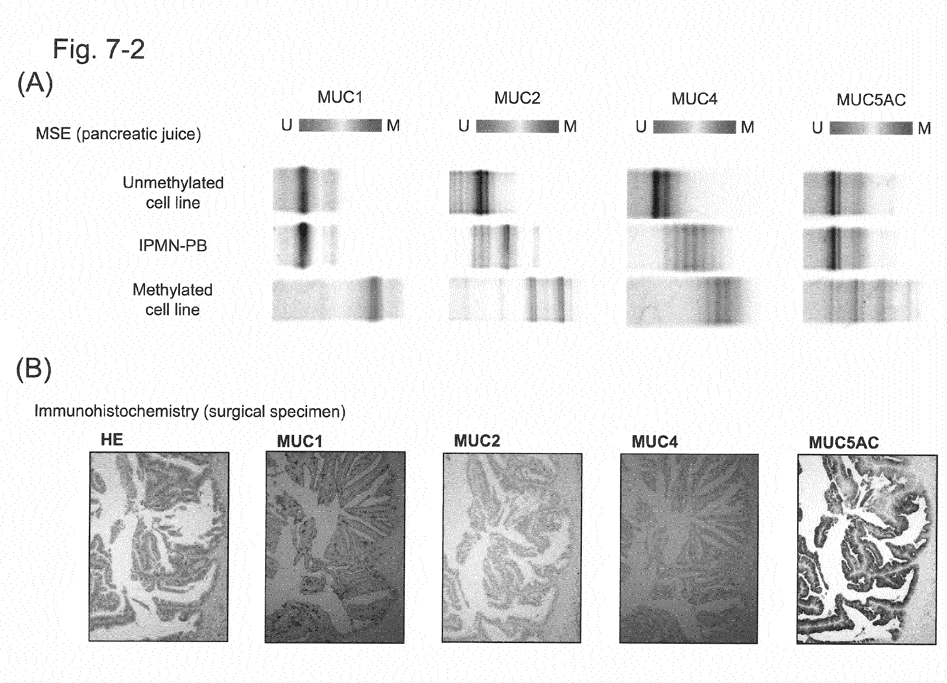

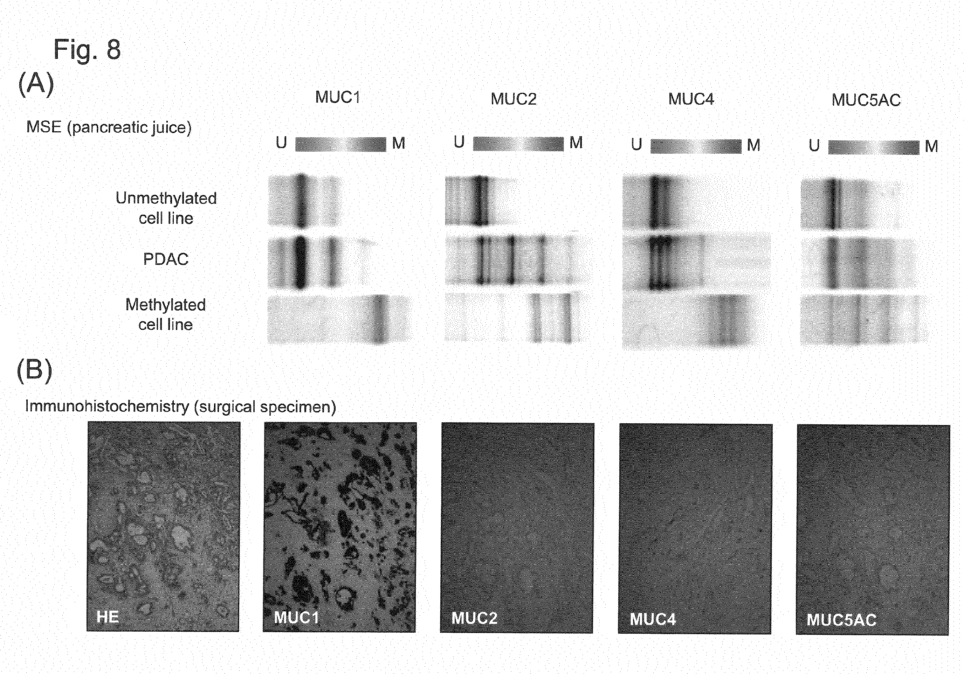

The results are shown in Figs. 5 to 8. Fig. 5 shows the results of methylation analysis and immunostaining of the IPMN-gastric type tumor. Fig. 6 (Figs. 6-1 and 6-2) each show the results of methylation analysis and immunostaining of the IPMN-intestinal type tumor. Fig. 7 (Figs. 7-1 and 7-2) each show the results of methylation analysis and immunostaining of the IPMN-PB type tumor. Fig. 8 shows the results of methylation analysis and immunostaining of PDAC. In Figs. 5 to 8, Panel (A) shows a photograph of a gel used in the MSE method, and Panel (B) shows a photograph showing the expression of proteins encoded by human MUC genes analyzed via immunohistochemical staining.

In Panel (A) shown in Figs. 5 to 8, the density gradient of the denaturing agent in a gel is adjusted to increase in the direction from the cathode to the anode of electrophoresis (from the left toward the right in the panel). Accordingly, "U" indicates unmethylation, "M" indicates methylation, and the color gradient from green to yellow to red indicates the degree of methylation, from unmethylation (0%) to methylation (100%).

In Panel (B) shown in Figs. 5 to 8, "HE" indicates the results of hematoxylin and eosin staining.

### 2-2. Results of statistical analysis based on emission intensity of band shown in photograph of gel used for MSE

Emission intensity levels of the bands in photographs showing gel after DGGE in the MSE method shown in Figs. 5 to 8 were quantified using ImageJ and subjected to statistical processing using "R" software for statistical analysis.

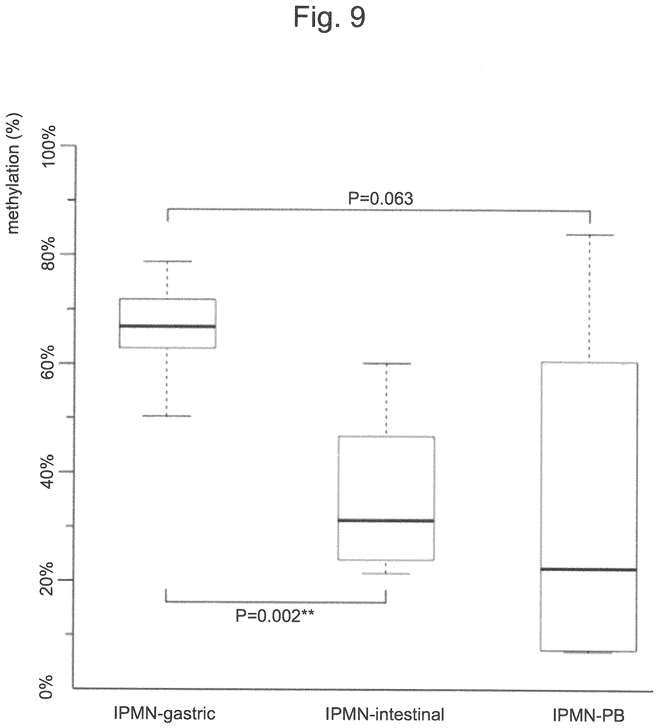

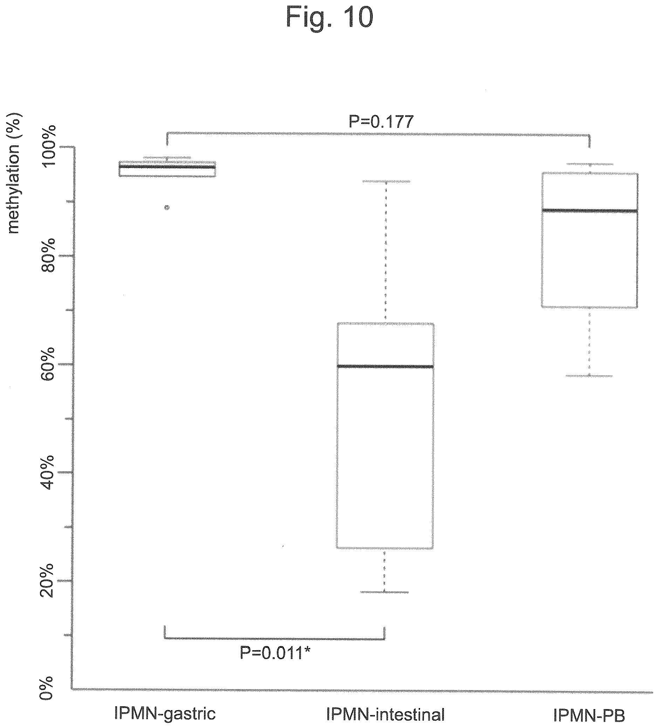

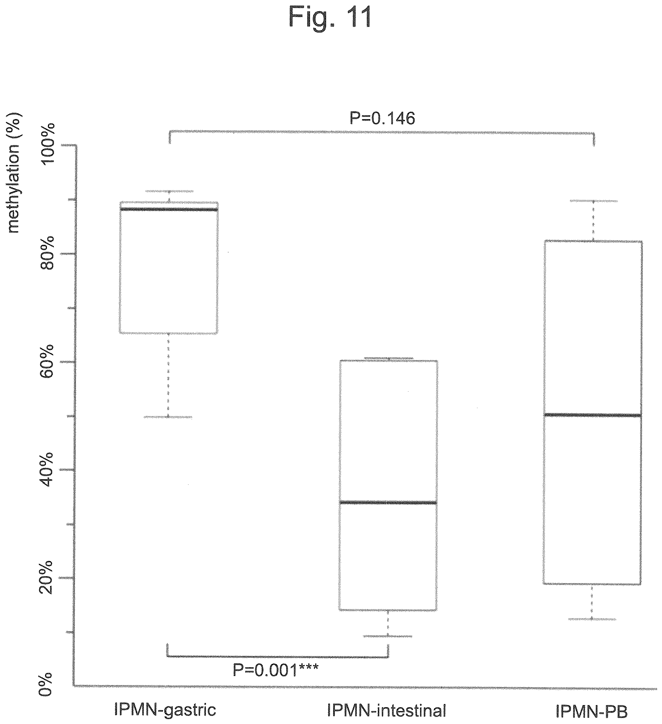

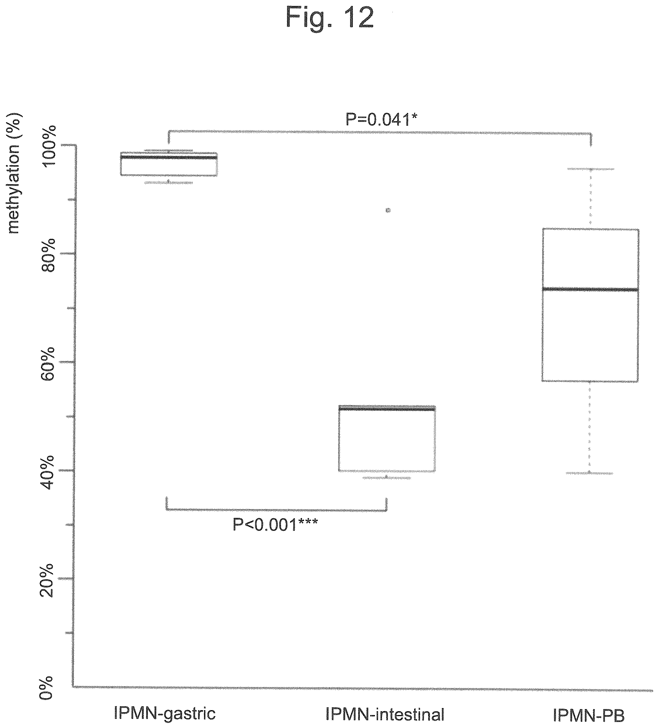

The results are shown in Figs. 9 to 12. Fig. 9 shows the results of statistical analysis of the MUC1 gene. Fig. 10 shows the results of statistical analysis of the MUC2 gene. Fig. 11 shows the results of statistical analysis of the MUC4 gene. Fig. 12 shows the results of statistical analysis of the MUC5AC gene. In Figs. 9 to 12, the vertical axis represents the methylated DNA content ratio (methylation %).

As is apparent from Figs. 9 to 12, all 4 types of mucin genes (i.e., the MUC1, MUC2, MUC4, and MUC5AC genes) are methylated in the case of the IPMN-gastric type tumor, and all of such 4 types of mucin genes are unmethylated in the case of the IPMN-intestinal type tumor. Significant differences are observed via statistical processing.

In addition, differences were observed in the methylation status of the MUC1 gene and the MUC5AC gene between the IPMN-gastric type tumor and the IPMN-PB type tumor.

### 2-3. Results of disease type prediction based on results of methylation analysis of 4 types of mucin genes

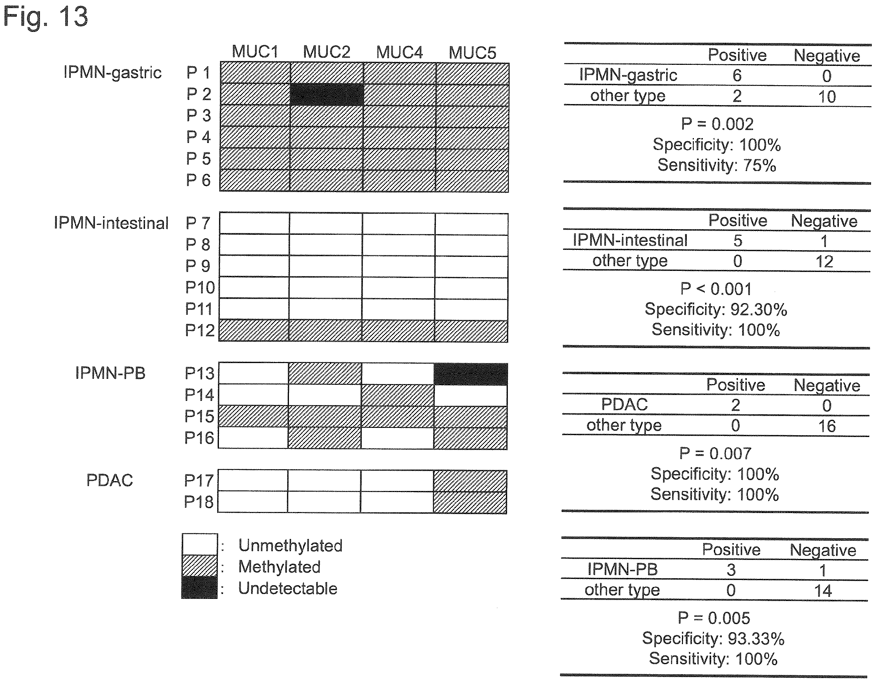

The results of disease type prediction based on the results of methylation analysis of the 4 above types of mucin genes are shown in Fig. 13. In the left panel of Fig. 13, "PX" (wherein X indicates a number from 1 to 18) represents a sample.

In Fig. 13, the methylation or unmethylation statuses of the genes were evaluated by designating the cut-off values at 50% (MUC1), 70% (MUC2), 50% (MUC4), and 60% (MUC5AC).

As shown in Fig. 13, disease type prediction was performed using such 4 types of mucin genes so as to identify the detected pancreatic tumor as: (1) IPMN-gastric if all 4 genes were methylated; (2) IPMN-intestinal if all 4 genes were unmethylated; (3) PDAC if the MUC1, MUC2, and MUC4 genes were unmethylated and the MUC5AC gene was methylated; or (4) IPMN-PB if none of the above were applicable. As a result, the results of prediction were found to be attained with high sensitivity and specificity. Accordingly, analysis of the methylation statuses of the 4 above types of genes contained in a pancreatic fluid by the MSE method is considered to provide information that is useful for disease type prediction.

In Example 1, methylated DNA content ratios are compared. When a band indicating unmethylation is observed as in the case of the MUC1 gene of IPMN-gastric tumor, but high methylated DNA content is observed as a whole, for example, the gene of interest can be determined as exhibiting a trend of methylation.

Pancreatic tumor malignancy is elevated in order from the gastric-type tumor, the intestinal-type tumor, the pancreatobiliary (PB)-type tumor, and to the pancreatic ductal adenocarcinoma (PDAC).

### [Example 2] Pancreatic tumor type prediction (2) through methylation analysis of 4 types of mucin genes

In Example 2, 4 types of mucin genes (i.e., the MUC1, MUC2, MUC4, and MUC5AC genes) in the pancreatic fluid samples of human pancreatic ductal adenocarcinoma (PDAC), an intraductal papillary mucinous neoplasm of the gastric type (IPMN-gastric), an intraductal papillary mucinous neoplasm of the intestinal type (IPMN-intestinal), and an intraductal papillary mucinous neoplasm of the pancreatobiliary type (IPMN-PB) were subjected to methylation analysis in the same manner as in Example 1, and disease type prediction was performed based on the results of methylation analysis. The methods of methylation analysis and disease type prediction were in accordance with those described in Example 1.

The samples subjected to analysis are shown in Table 10 below.

**Table 10**

| | |
|---|---|
| Gastric-type IPMN | 7 samples |
| Intestinal-type IPMN | 8 samples |
| P.B.-type IPMN | 5 samples |
| PDAC | 2 samples |
| other | 3 samples |
| Total | 25 samples |

"Other" samples are those of diseases other than the gastric-type, the intestinal-type, the PB-type, and PDAC tumors.

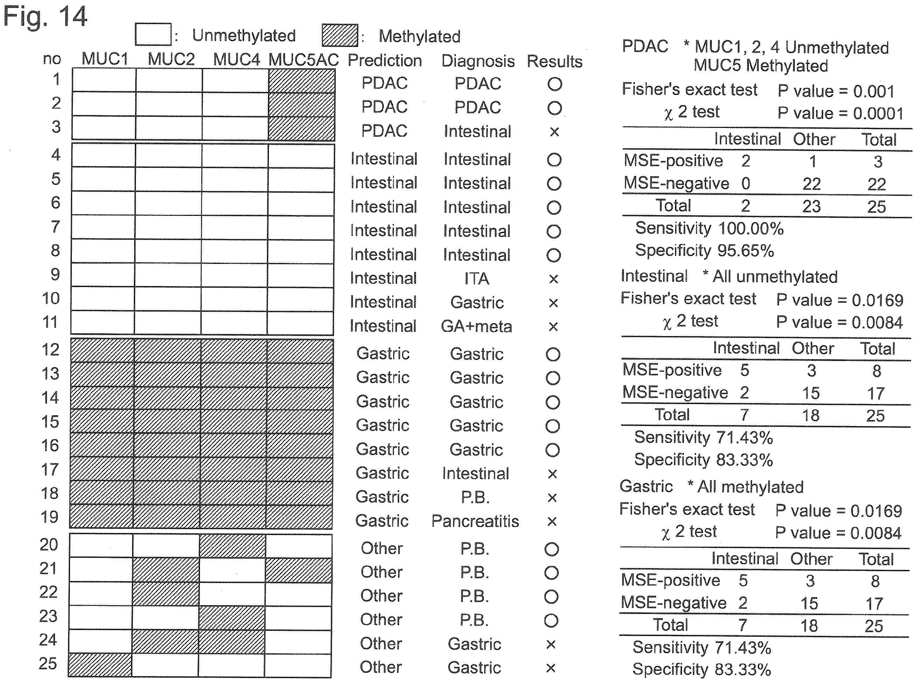

Fig. 14 shows the results of disease type prediction based on the results of methylation analysis of the 4 above types of mucin genes. In the left panel of Fig. 14, each number represents a sample number.

As shown in Fig. 14, disease type prediction was performed using the 4 types of mucin genes under the following conditions: (1) IPMN-gastric if all 4 genes were methylated; (2) IPMN-intestinal if all 4 genes were unmethylated; (3) PDAC if the MUC1, MUC2, and MUC4 genes were unmethylated and the MUC5AC gene was methylated; or (4) IPMN-PB if none of the above were applicable. As a result, the results of prediction were found to be attained with high sensitivity and specificity.

### Industrial Applicability

According to the present disclosure, pancreatic tumor type can be diagnosed at an early stage with the use of a pancreatic fluid sample that can be obtained from a subject in a less invasive manner.

## Claims

1. An examination method for determining pancreatic tumor type comprising:
a first step of detecting the degree of methylation in a 5'-untranslated region or a region comprising a 5'-untranslated region and a translated region of a gene encoding a mucin core protein in a set of genes including the MUC1 gene, the MUC2 gene, the MUC4 gene, and the MUC5AC gene in a pancreatic fluid sample from a subject, wherein the 5'-untranslated region or the region comprising a 5'-untranslated region and a translated region in each of the MUC1 gene, the MUC2 gene, the MUC4 gene, and the MUC5AC gene comprises the nucleotide sequence as shown in SEQ ID NOs: 1 to 4, respectively; and
a second step of identifying a pancreatic tumor, using the degree of methylation as an index value, as being of pancreatic tumor type selected from the group consisting of pancreatic ductal adenocarcinoma, an intraductal papillary mucinous neoplasm of the gastric type, and an intraductal papillary mucinous neoplasm of the intestinal type, wherein the methylated or unmethylated statuses of the genes are evaluated by designating cut-off values for methylated DNA content as 50% for the MUC1 gene, 70% for the MUC2 gene, 50% for the MUC4 gene and 60% for the MUC5AC gene and, the pancreatic tumor is identified as:
(a) an intraductal papillary mucinous neoplasm of the gastric type when the 5'-untranslated regions or the regions comprising 5'-untranslated regions and translated regions of the MUC1 gene, the MUC2 gene, the MUC4 gene, and the MUC5AC gene are methylated;
(b) an intraductal papillary mucinous neoplasm of the intestinal type when the 5'-untranslated regions or the regions comprising 5'-untranslated regions and translated regions of the MUC1 gene, the MUC2 gene, the MUC4 gene, and the MUC5AC gene are unmethylated; or
(c) a pancreatic ductal adenocarcinoma when the 5'-untranslated regions or the regions comprising 5'-untranslated regions and translated regions of the MUC1 gene, the MUC2 gene, and the MUC4 gene are unmethylated and the 5'-untranslated region or the region comprising a 5'-untranslated region and a translated region of the MUC5AC gene is methylated.

2. The method according to claim 1, wherein the first step comprises steps of:
subjecting DNA obtained from a pancreatic fluid sample to bisulfite treatment;
subjecting the DNA after bisulfite treatment to a first PCR using a first set of primers corresponding to outer regions of a 5'-untranslated region or a region comprising a 5'-untranslated region and a translated region of the gene encoding a mucin core protein;
subjecting the DNA amplified via the first PCR to a second PCR using a second set of primers corresponding to the 5'-untranslated region or the region comprising a 5'-untranslated region and a translated region of the gene encoding a mucin core protein; and
subjecting the DNA amplified via the second PCR to denaturing gradient gel electrophoresis, with the annealing positions of the second set of primers being located inside the annealing positions of the first set of primers relative to the 5'-untranslated region or the region comprising a 5'-untranslated region and a translated region of the gene encoding a mucin core protein.

3. The method according to claim 2, wherein one of a pair of primers constituting the second set of primers has a GC-clamp sequence in its 5' side.

4. The method according to claim 2 or 3, wherein the density gradient of the denaturing gradient gel is limited to a denaturing density gradient.

5. Use of an examination kit for implementing a method according to any one of claims 1 to 4 for determining pancreatic tumor type, comprising:
a set of primers used for implementing the method according to any one of claims 1 to 4; and
an instruction for determining pancreatic tumor type describing identification of a pancreatic tumor as:
(a) an intraductal papillary mucinous neoplasm of the gastric type when the 5'-untranslated regions or the regions comprising 5'-untranslated regions and translated regions of the MUC1 gene, the MUC2 gene, the MUC4 gene, and the MUC5AC gene are methylated;
(b) an intraductal papillary mucinous neoplasm of the intestinal type when the 5'-untranslated regions or the regions comprising 5'-untranslated regions and translated regions of the MUC1 gene, the MUC2 gene, the MUC4 gene, and the MUC5AC gene are unmethylated; or
(c) a pancreatic ductal adenocarcinoma when the 5'-untranslated regions or the regions comprising 5'-untranslated regions and translated regions of the MUC1 gene, the MUC2 gene, and the MUC4 gene are unmethylated and the 5'-untranslated region or the region comprising a 5'-untranslated region and a translated region of the MUC5AC gene is methylated.

6. The use according to claim 5, which comprises a first set of primers corresponding to outer regions of the 5'-untranslated region or the region comprising a 5'-untranslated region and a translated region of a gene encoding a mucin core protein and a second set of primers corresponding to the 5'-untranslated region or the region comprising a 5'-untranslated region and a translated region of a gene encoding a mucin core protein, with the annealing positions of the second set of primers being located inside the annealing positions of the first set of primers relative to the 5'-untranslated region or a region comprising a 5'-untranslated region and a translated region of a gene encoding a mucin core protein.

7. The use according to claim 5 or 6, which comprises a set of primers consisting of the nucleotide sequences as shown in SEQ ID NOs: 14 to 29, respectively.

## Patentansprüche

1. Untersuchungsverfahren zur Bestimmung eines Pankreastumortyps, umfassend:
einen ersten Schritt des Nachweises des Methylierungsgrads in einer 5'-untranslatierten Region oder einer Region, die eine 5'-untranslatierte Region und eine translatierte Region umfasst, eines Gens, das ein Mucin-Kernprotein codiert, in einem Set von Genen, das das MUC1-Gen, das MUC2-Gen, das MUC4-Gen und das MUC5AC-Gen in einer Pankreasflüssigkeitsprobe von einem Individuum enthält, wobei die 5'-untranslatierte Region oder die Region, die eine 5'-untranslatierte Region und eine translatierte Region umfasst, in jedem MUC1-Gen, MUC2-Gen, MUC4-Gen und MUC5AC-Gen respektive eine wie in SEQ ID NOs:1 bis 4 gezeigte Nucleotidsequenz umfasst; und
einen zweiten Schritt der Identifizierung eines Pankreastumors unter Verwendung des Grades der Methylierung als Indikatorwert, als Pankreastumortyp, der ausgewählt ist aus der Gruppe bestehend aus duktalem Adenokarzinom des Pankreas, intraduktaler papillär-muzinöser Neoplasie des gastrischen Typs und intraduktaler papillär-muzinöser Neoplasie des intestinalen Typs, wobei der methylierte oder nicht-methylierte Zustand der Gene durch Festlegen von Cut-off-Werten für den Gehalt an methylierter DNA als 50% für das MUC1-Gen, 70% für das MUC2-Gen, 50% für das MUC4-Gen und 60% für das MUC5AC-Gen bewertet wird und der Pankreastumor identifiziert wird als:
(a) eine intraduktale papillär-muzinöse Neoplasie des gastrischen Typs, wenn die 5'-untranslatierten Regionen oder die Regionen, die 5'-untranslatierte Regionen und translatierte Regionen umfassen, des MUC1-Gens, des MUC2-Gens, des MUC4-Gens und des MUC5AC-Gens, methyliert sind;
(b) eine intraduktale papillär-muzinöse Neoplasie des intestinalen Typs, wenn die 5'-untranslatierten Regionen oder die Regionen, die 5'-untranslatierte Regionen und translatierte Regionen umfassen, des MUC1-Gens, des MUC2-Gens, des MUC4-Gens und des MUC5AC-Gens, nicht methyliert sind; oder
(c) ein duktales Adenokarzinom des Pankreas, wenn die 5'-untranslatierten Regionen oder die Regionen, die 5'-untranslatierte Regionen und translatierte Regionen umfassen, des MUC1-Gens, des MUC2-Gens und des MUC4-Gens, nicht methyliert sind, und die 5'-untranslatierte Region oder die Region, die eine 5'-untranslatierte Region und eine translatierte Region umfasst, des MUC5AC-Gens, methyliert ist.

2. Verfahren nach Anspruch 1, wobei der erste Schritt die Schritte umfasst:
Unterziehen der aus einer Pankreasflüssigkeitsprobe erhaltenen DNA einer Bisulfit-Behandlung;
nach der Bisulfit-Behandlung Unterziehen der DNA einer ersten PCR unter Verwendung eines ersten Primersets, das äußeren Regionen einer 5'-untranslatierten Region oder einer Region entspricht, die eine 5'-untranslatierte Region und eine translatierte Region umfasst, des Gens, das ein Mucin-Kernprotein codiert;
Unterziehen der mittels der ersten PCR amplifizierten DNA einer zweiten PCR unter Verwendung eines zweiten Primersets, das der 5'-untranslatierten Region oder der Region entspricht, die eine 5'-untranslatierte Region und eine translatierte Region umfasst, des Gens, das ein Mucin-Kernprotein codiert; und
Unterziehen der mittels der zweiten PCR amplifizierten DNA einer denaturierenden Gradienten-Gelelektrophorese, wobei die Annealing-Positionen des zweiten Primersets im Verhältnis zu der 5'-untranslatierten Region oder der Region, die eine 5'-untranslatierte Region und eine translatierte Region umfasst, des Gens, das ein Mucin-Kernprotein codiert, innerhalb der Annealing-Positionen des ersten Primersets lokalisiert sind.

3. Verfahren nach Anspruch 2, wobei einer der Primer eines Primerpaars, das das zweite Primerset bildet, in seinem 5'-Ende eine GC-Klammer-Sequenz aufweist.

4. Verfahren nach Anspruch 2 oder 3, wobei der Dichtegradient des Denaturierungsgradientengels auf einen Denaturierungsdichtegradienten beschränkt ist.

5. Verwendung eines Untersuchungskits zur Implementierung eines Verfahrens nach einem der Ansprüche 1 bis 4 zur Bestimmung eines Pankreastumortyps, umfassend:
ein Primerset, das zur Implementierung des Verfahrens nach einem der Ansprüche 1 bis 4 verwendet wird; und
eine Anleitung für die Bestimmung des Pankreastumortyps, das die Identifizierung eines Pankreastumors beschreibt als:
(a) eine intraduktale papillär-muzinöse Neoplasie des gastrischen Typs, wenn die 5'-untranslatierten Regionen oder die Regionen, die 5'-untranslatierte Regionen und translatierte Regionen umfassen, des MUC1-Gens, des MUC2-Gens, des MUC4-Gens und des MUC5AC-Gens, methyliert sind;
(b) eine intraduktale papillär-muzinöse Neoplasie des intestinalen Typs, wenn die 5'-untranslatierten Regionen oder die Regionen, die 5'-untranslatierte Regionen und translatierte Regionen umfassen, des MUC1-Gens, des MUC2-Gens, des MUC4-Gens und des MUC5AC-Gens, nicht methyliert sind; oder
(c) ein duktales Adenokarzinom des Pankreas, wenn die 5'-untranslatierten Regionen oder die Regionen, die 5'-untranslatierte Regionen und translatierte Regionen umfassen, des MUC1-Gens, des MUC2-Gens und des MUC4-Gens, nicht methyliert sind, und die 5'-untranslatierte Region oder die Region, die eine 5'-untranslatierte Region und eine translatierte Region umfasst, des MUC5AC-Gens, methyliert ist.

6. Verwendung nach Anspruch 5, umfassend ein erstes Primerset, das äußeren Regionen der 5'-untranslatierten Region oder der Region entspricht, die eine 5'-untranslatierte Region und eine translatierte Region umfasst, eines Gens, das ein Mucin-Kernprotein codiert, und ein zweites Primerset, das der 5'-untranslatierten Region oder der Region entspricht, die eine 5'-untranslatierte Region und eine translatierte Region umfasst, eines Gens, das ein Mucin-Kernprotein codiert, wobei die Annealing-Positionen des zweiten Primersets im Verhältnis zu der 5'-untranslatierten Region oder einer Region, die eine 5'-untranslatierte Region und eine translatierte Region umfasst, eines Gens, das ein Mucin-Kernprotein codiert, innerhalb der Annealing-Positionen des ersten Primersets lokalisiert sind.

7. Verwendung nach Anspruch 5 oder 6, umfassend ein Primerset, das respektive aus den wie in SEQ ID NOs:14 bis 29 gezeigten Nucleotidsequenzen besteht.

## Revendications

1. Méthode d'examen pour déterminer le type de tumeur pancréatique comprenant :
une première étape de détection du degré de méthylation dans une région 5'-non traduite ou une région comprenant une région 5'-non traduite et une région traduites d'un gène codant pour une protéine du coeur de la mucine dans un ensemble de gènes comprenant le gène MUC1, le gène MUC2, le gène MUC4, et le gène MUC5AC dans un échantillon de fluide pancréatique provenant d'un sujet, dans laquelle la région 5'-non traduite ou la région comprenant une région 5'-non traduite et une région traduite dans chacun des gènes MUC1, MUC2, MUC4, et MUC5AC comprend la séquence nucléotidique telle que représentée par SEQ ID NOs : 1 à 4, respectivement ; et
une seconde étape d'identification d'une tumeur pancréatique, en utilisant le degré de méthylation comme une valeur d'indice, comme étant d'un type de tumeur pancréatique sélectionné dans le groupe constitué par l'adénocarcinome canalaire pancréatique, une néoplasie intracanalaire papillaire mucineuse de type gastrique, et une néoplasie intracanalaire papillaire mucineuse de type intestinal, dans laquelle les états méthylés ou non méthylés des gènes sont évalués en désignant des valeurs seuil pour la teneur en ADN méthylé de 50 % pour le gène MUC1, 70 % pour le gène MUC2, 50 % pour le gène MUC4 et 60 % pour le gène MUC5AC et, la tumeur pancréatique est identifiée comme :
(a) une néoplasie intracanalaire papillaire mucineuse de type gastrique lorsque les régions 5'-non traduites ou les régions comprenant des régions 5'-non traduites et des régions traduites du gène MUC 1, du gène MUC2, du gène MUC4, et du gène MUC5AC sont méthylées ;
(b) une néoplasie intracanalaire papillaire mucineuse de type intestinal lorsque les régions 5'-non traduites ou les régions comprenant des régions 5'-non traduites et des régions traduites du gène MUC1, du gène MUC2, du gène MUC4, et du gène MUC5AC sont non méthylées ; ou
(c) un adénocarcinome canalaire pancréatique lorsque les régions 5'-non traduites ou les régions comprenant des régions 5'-non traduites et des régions traduites du gène MUC1, du gène MUC2, et du gène MUC4 sont non méthylées et la région 5'-non traduite ou la région comprenant une région 5'-non traduite et une région traduite du gène MUC5AC est méthylée.

2. Méthode selon la revendication 1, dans laquelle la première étape comprend des étapes de :
soumission de l'ADN obtenu à partir d'un échantillon de fluide pancréatique à un traitement au bisulfite ;
soumission de l'ADN après traitement au bisulfite à une première PCR en utilisant un premier ensemble d'amorces correspondant aux régions externes d'une région 5'-non traduite ou d'une région comprenant une région 5'-non traduite et une région traduite du gène codant pour une protéine du coeur de la mucine ;
soumission de l'ADN amplifié via la première PCR à une seconde PCR en utilisant un second ensemble d'amorces correspondant à la région 5'-non traduite ou à la région comprenant une région 5'-non traduite et une région traduite du gène codant pour une protéine du coeur de la mucine ; et
soumission de l'ADN amplifié via la seconde PCR à une électrophorèse sur gel en gradient dénaturant, avec les positions d'hybridation du second ensemble d'amorces localisées à l'intérieur des positions d'hybridation du premier ensemble d'amorces par rapport à la région 5'-non traduite ou à la région comprenant une région 5'-non traduite et une région traduite du gène codant pour une protéine du coeur de la mucine.

3. Méthode selon la revendication 2, dans laquelle l'une d'une paire d'amorces constituant le second ensemble d'amorces comporte une séquence de type GC-clamp sur son extrémité 5'.

4. Méthode selon la revendication 2 ou 3, dans laquelle le gradient de densité du gel en gradient dénaturant est limité à un gradient de densité dénaturant.

5. Utilisation d'un kit d'examen pour la mise en oeuvre d'une méthode selon l'une quelconque des revendications 1 à 4 pour déterminer le type de tumeur pancréatique, comprenant :
un ensemble d'amorces utilisé pour la mise en oeuvre de la méthode selon l'une quelconque des revendications 1 à 4 ; et
une instruction pour déterminer le type de tumeur pancréatique décrivant l'identification d'une tumeur pancréatique comme :
(a) une néoplasie intracanalaire papillaire mucineuse de type gastrique lorsque les régions 5'-non traduites ou les régions comprenant des régions 5'-non traduites et des régions traduites du gène MUC1, du gène MUC2, du gène MUC4, et du gène MUC5AC sont méthylées ;
(b) une néoplasie intracanalaire papillaire mucineuse de type intestinal lorsque les régions 5'-non traduites ou les régions comprenant des régions 5'-non traduites et des régions traduites du gène MUC1, du gène MUC2, du gène MUC4, et du gène MUC5AC sont non méthylées ; ou
(c) un adénocarcinome canalaire pancréatique lorsque les régions 5'-non traduites ou les régions comprenant des régions 5'-non traduites et des régions traduites du gène MUC1, du gène MUC2, et du gène MUC4 sont non méthylées et la région 5'-non traduite ou la région comprenant une région 5'-non traduite et une région traduite du gène MUC5AC est méthylée.

6. Utilisation selon la revendication 5, qui comprend un premier ensemble d'amorces correspondant aux régions externes de la région 5'-non traduite ou de la région comprenant une région 5'-non traduite et une région traduite d'un gène codant pour une protéine du coeur de la mucine et un second ensemble d'amorces correspondant à la région 5'-non traduite ou à la région comprenant une région 5'-non traduite et une région traduite d'un gène codant pour une protéine du coeur de la mucine, avec les positions d'hybridation du second ensemble d'amorces localisées à l'intérieur des positions d'hybridation du premier ensemble d'amorces par rapport à la région 5'-non traduite ou à la région comprenant une région 5'-non traduite et une région traduite d'un gène codant pour une protéine du coeur de la mucine.

7. Utilisation selon la revendication 5 ou 6, qui comprend un ensemble d'amorces constitué des séquences nucléotidiques telles que représentées par les SEQ ID NOs : 14 à 29, respectivement.


### SEQUENCE LISTING

<110> Kagoshima University
<120> A Method for Diagnosing Types of Pancreatic Tumor
<130> PH-5292-PCT
<150> JP 2011-144847
   <151> 2011-06-29
<160> 29
<170> Patent In version 3.4
<210> 1
   <211> 126
   <212> DNA
   <213> Homo sapiens
<400> 1
   gggggcgggg ttttgtcacc tgtcacctgc tccggctgtg cctagggcgg gcgggcgggg 60
   agtgggggga ccggtataaa gcggtaggcg cctgtgcccg ctccacctct caagcagcca 120
   gcgcct 126
<210> 2
   <211> 175
   <212> DNA
   <213> Homo sapiens
<400> 2
   gaatgggttg gtcctcccag cgtaacgtga gcctgatcag gcctggggac ctgctcagcg 60
   ggtgtctggg ggcccatggc gggctaagga gcctgaccag acttgcttct ggcaggacac 120
   ccctcccccg gccaccctgg gctcgcccct ctagtagctg catgtgttcc ccggg 175
<210> 3
   <211> 186
   <212> DNA
   <213> Homo sapiens
<400> 3
   gcgtggggcc ccgccctctt ttgtcctctt cccaggttcc ctggcccctt cggagaaacg 60
   cacttggttc gggccagccg cctgagggga cgggctcacg tctgctcctc acactgcagc 120
   tgctgggccg tggagcttcc ccagggagcc agggggactt ttgccgcagc catgaagggg 180
   gcacgc 186
<210> 4
   <211> 145
   <212> DNA
   <213> Homo sapiens
<400> 4
   gtgcctgagc ccaggcccca agaggaagcc cagcacagcc aggggtcacc aacactggtg 60
   gggggaagtc accccagctg gaccccagca gcggccctgg gtgacgtctg gctgagggag 120
   gagaaagctg tggctggggc ggcaa 145
<210> 5
   <211> 451
   <212> DNA
   <213> Homo sapiens
<400> 5
   cccctccccc ggagccaggg agtggttggt gaaaggggga ggccagctgg agaacaaacg      60
   ggtagtcagg gggttgagcg attagagccc ttgtacccta cccaggaatg gttggggagg     120
   aggaggaaga ggtaggaggt aggggagggg gcggggtttt gtcacctgtc acctgctccg     180
   gctgtgccta gggcgggcgg gcggggagtg gggggaccgg tataaagcgg taggcgcctg     240
   tgcccgctcc acctctcaag cagccagcgc ctgcctgaat ctgttctgcc ccctccccac     300
   ccatttcacc accaccatga caccgggcac ccagtctcct ttcttcctgc tgctgctcct     360
   cacagtgctt acaggtgagg ggcacgaggt ggggagtggg ctgccctgct taggtggtct     420
   tcgtggtctt tctgtgggtt ttgctccctg g                                    451
<210> 6
   <211> 451
   <212> DNA
   <213> Artificial
<220>
   <223> A promoter region and a flanking coding region of human MUC1 gene which are bisulfite-treated, showing the urasil to which the unmethylated cytosine is converted as thymine
<400> 6
   tttttttttt ggagttaggg agtggttggt gaaaggggga ggttagttgg agaataaatg 60
   ggtagttagg gggttgagtg attagagttt ttgtatttta tttaggaatg gttggggagg 120
   aggaggaaga ggtaggaggt aggggagggg gtggggtttt gttatttgtt atttgttttg 180
   gttgtgttta gggtgggtgg gtggggagtg gggggattgg tataaagtgg taggtgtttg 240
   tgtttgtttt attttttaag tagttagtgt ttgtttgaat ttgttttgtt ttttttttat 300
   ttattttatt attattatga tattgggtat ttagtttttt ttttttttgt tgttgttttt 360
   tatagtgttt ataggtgagg ggtatgaggt ggggagtggg ttgttttgtt taggtggttt 420
   ttgtggtttt tttgtgggtt ttgttttttg g 451
<210> 7
   <211> 560
   <212> DNA
   <213> Homo sapiens
<400> 7
   gaaggatccc acatcctccc tgccctcggg gaggcccctt tctggggtca ggcctggaag 60
   ctgccccaga gcttgggccc caggaatggg ttggtcctcc cagcgtaacg tgagcctgat 120
   caggcctggg gacctgctca gcgggtgtct gggggcccat ggcgggctaa ggagcctgac 180
   cagacttgct tctggcagga cacccctccc ccggccaccc tgggctcgcc cctctagtag 240
   ctgcatgtgt tccccgggtg tgtgttggca ttcaggctac agggctgcct catcctgaag 300
   aaggctgcgt ttacccaggg agccataaag agatgacctc cgataacctg aatcaatatt 360
   tccccattgg ggctcgggcc cccgcagctg tcttcttgat catctggcag atgccacacc 420
   cacccttggc cctcccctgc cttcctgccc tcctaccctc ctgccaggac atataaggac 480
   cagacccctg cccccgggcg caacccacac cgcccctgcc agccaccatg ggctgccacc 540
   tagcccgcct ggcggctgtg 560
<210> 8
   <211> 560
   <212> DNA
   <213> Artificial
<220>
   <223> A promoter region and a flanking coding region of human MUC2 gene which are bisulfite-treated, showing the urasil to which the unmethylated cytosine is converted as thymine
<400> 8
   gaaggatttt atattttttt tgtttttggg gaggtttttt tttggggtta ggtttggaag    60
   ttgttttaga gtttgggttt taggaatggg ttggtttttt tagtgtaatg tgagtttgat   120
   taggtttggg gatttgttta gtgggtgttt gggggtttat ggtgggttaa ggagtttgat   180
   tagatttgtt tttggtagga tatttttttt ttggttattt tgggtttgtt tttttagtag   240
   ttgtatgtgt tttttgggtg tgtgttggta tttaggttat agggttgttt tattttgaag   300
   aaggttgtgt ttatttaggg agttataaag agatgatttt tgataatttg aattaatatt   360
   tttttattgg ggtttgggtt tttgtagttg tttttttgat tatttggtag atgttatatt   420
   tatttttggt ttttttttgt ttttttgttt ttttattttt ttgttaggat atataaggat   480
   tagatttttg tttttgggtg taatttatat tgtttttgtt agttattatg gggttgttat   540
   tagtttgttt ggtggttgtg                                               560
<210> 9
   <211> 345
   <212> DNA
   <213> Homo sapiens
<400> 9
   cagagcaacg ggtgtatcgg tgttttcttt cctggtgggg tagtggggtg gggctgagga 60
   gagaaaaggg tgattagcgt ggggccccgc cctcttttgt cctcttccca ggttccctgg 120
   ccccttcgga gaaacgcact tggttcgggc cagccgcctg aggggacggg ctcacgtctg 180
   ctcctcacac tgcagctgct gggccgtgga gcttccccag ggagccaggg ggacttttgc 240
   cgcagccatg aagggggcac gctggaggag ggtcccctgg gtgtccctga gctgcctgtg 300
   tctctgcctc cttccgcatg tggtcccagg taagtgatgg agaca 345
<210> 10
   <211> 345
   <212> DNA
   <213> Artificial
<220>
   <223> A promoter region and a flanking coding region of human MUC4 gene which are bisulfite-treated, showing the urasil to which the unmethylated cytosine is converted as thymine
<400> 10
   tagagtaatg ggtgtattgg tgtttttttt tttggtgggg tagtggggtg gggttgagga 60
   gagaaaaggg tgattagtgt ggggttttgt ttttttttgt ttttttttta ggttttttgg 120
   tttttttgga gaaatgtatt tggtttgggt tagttgtttg aggggatggg tttatgtttg 180
   ttttttatat tgtagttgtt gggttgtgga gtttttttag ggagttaggg ggatttttgt 240
   tgtagttatg aagggggtat gttggaggag ggttttttgg gtgtttttga gttgtttgtg 300
   tttttgtttt tttttgtatg tggttttagg taagtgatgg agata 345
<210> 11
   <211> 700
   <212> DNA
   <213> Homo sapiens
<400> 11
   ccaggaggca ggtggctgcg ccacggctcg gccagccctg cctgcaccct ctgagagccc 60
   ccagcaaggc tgcagtgacc acctcagatt cccctctgag gcctgtggcg tggccgggac 120
   ctcactgccc ctggggacac acagaaaatg cccacagagc tcagaaacaa ggcccagtgg 180
   gttttctgga aagttctggg tgtgtggagc ctggggctgt aggctctgga actgtaggca 240
   ctggcttcag gcctcctgag gcctcggcct ggtggggttt tcatgggacc aggtggtcag 300
   cccgtggccc atgcccaggg gttttgggtg cctgagccca ggccccaaga ggaagcccag 360
   cacagccagg ggtcaccaac actggtgggg ggaagtcacc ccagctggac cccagcagcg 420
   gccctgggtg acgtctggct gagggaggag aaagctgtgg ctggggcggc aaggcctggg 480
   tggccagttg gccaggtgcc ccggggcttg gcccagcctc agacacgcag ggggcactcc 540
   cctctgaggg ccacgctggt gactcagact gttcagaggt cacggtatgg actgggccag 600
   tgactcaggc ctgtcctctg ttgggggctg gacactgact cacccactgc ctcctgtcta 660
   tctgagggcg taaggagggc aggccttcag gcactcacat 700
<210> 12
   <211> 700
   <212> DNA
   <213> Artificial
<220>
   <223> A promoter region of human MUC5AC gene which is bisulfite-treated, showing the urasil to which the unmethylated cytosine is converted as thymine
<400> 12
   ttaggaggta ggtggttgtg ttatggtttg gttagttttg tttgtatttt ttgagagttt      60
   ttagtaaggt tgtagtgatt attttagatt tttttttgag gtttgtggtg tggttgggat     120
   tttattgttt ttggggatat atagaaaatg tttatagagt ttagaaataa ggtttagtgg     180
   gttttttgga aagttttggg tgtgtggagt ttggggttgt aggttttgga attgtaggta    240
   ttggttttag gttttttgag gttttggttt ggtggggttt ttatgggatt aggtggttag     300
   tttgtggttt atgtttaggg gttttgggtg tttgagttta ggttttaaga ggaagtttag    360
   tatagttagg ggttattaat attggtgggg ggaagttatt ttagttggat tttagtagtg    420
   gttttgggtg atgtttggtt gagggaggag aaagttgtgg ttggggtggt aaggtttggg    480
   tggttagttg gttaggtgtt ttggggtttg gtttagtttt agatatgtag ggggtatttt    540
   tttttgaggg ttatgttggt gatttagatt gtttagaggt tatggtatgg attgggttag    600
   tgatttaggt ttgttttttg ttgggggttg gatattgatt tatttattgt tttttgttta   660
   tttgagggtg taaggagggt aggtttttag gtatttatat                          700
<210> 13
   <211> 40
   <212> DNA
   <213> Artificial
<220>
   <223> GC-clamp
<400> 13
   cgcccgccgc gcgcggcggg cggggcgggg gcacgggggg 40
<210> 14
   <211> 20
   <212> DNA
   <213> Artificial
<220>
   <223> Primer
<400> 14
   aaagggggag gttagttgga 20
<210> 15
   <211> 20
   <212> DNA
   <213> Artificial
<220>
   <223> Primer
<400> 15
   aaacaaccca ctccccacct 20
<210> 16
   <211> 60
   <212> DNA
   <213> Artificial
<220>
   <223> Primer
<400> 16
   cgcccgccgc gcgcggcggg cggggcgggg gcacgggggg aagaggtagg aggtagggga 60
<210> 17
   <211> 20
   <212> DNA
   <213> Artificial
<220>
   <223> Primer
<400> 17
   aaaacaaaac aaattcaaac 20
<210> 18
   <211> 20
   <212> DNA
   <213> Artificial
<220>
   <223> Primer
<400> 18
   tttggggtta ggtttggaag 20
<210> 19
   <211> 23
   <212> DNA
   <213> Artificial
<220>
   <223> Primer
<400> 19
   accttcttca aaataaaaca acc 23
<210> 20
   <211> 60
   <212> DNA
   <213> Artificial
<220>
   <223> Primer
<400> 20
   cgcccgccgc gcgcggcggg cggggcgggg gcacgggggg ttttagagtt tgggttttag 60
<210> 21
   <211> 21
   <212> DNA
   <213> Artificial
<220>
   <223> Primer
<400> 21
   taacctaaat accaacacac a 21
<210> 22
   <211> 17
   <212> DNA
   <213> Artificial
<220>
   <223> Primer
<400> 22
   tagtggggtg gggttga 17
<210> 23
   <211> 17
   <212> DNA
   <213> Artificial
<220>
   <223> Primer
<400> 23
   aaacacccaa aaaaccc 17
<210> 24
   <211> 60
   <212> DNA
   <213> Artificial
<220>
   <223> Primer
<400> 24
   cgcccgccgc gcgcggcggg cggggcgggg gcacgggggg aggagagaaa agggtgatta 60
<210> 25
   <211> 20
   <212> DNA
   <213> Artificial
<220>
   <223> Primer
<400> 25
   acccaaaaaa ccctcctcca 20
<210> 26
   <211> 20
   <212> DNA
   <213> Artificial
<220>
   <223> Primer
<400> 26
   aaagttttgg gtgtgtggag 20
<210> 27
   <211> 23
   <212> DNA
   <213> Artificial
<220>
   <223> Primer
<400> 27
   taaatcaata tccaaccccc aac 23
<210> 28
   <211> 60
   <212> DNA
   <213> Artificial
<220>
   <223> Primer
<400> 28
   cgcccgccgc gcgcggcggg cggggcgggg gcacgggggg tttatgttta ggggttttgg 60
<210> 29
   <211> 20
   <212> DNA
   <213> Artificial
<220>
   <223> Primer
<400> 29
   accaactaac cacccaaacc 20